(19) Europäisches Patentamt
European Patent Office
Office européen des brevets

(11) **EP 3 867 003 B1**

(12) **EUROPEAN PATENT SPECIFICATION**

(45) Date of publication and mention
of the grant of the patent:
**28.08.2024 Bulletin 2024/35**

(21) Application number: **19817868.3**

(22) Date of filing: **14.11.2019**

(51) International Patent Classification (IPC):
***B23K 26/0622*** (2014.01)  ***B23K 26/53*** (2014.01)

(52) Cooperative Patent Classification (CPC):
**B23K 26/0624; B23K 26/53;** B23K 2103/42

(86) International application number:
**PCT/US2019/061451**

(87) International publication number:
**WO 2020/102514 (22.05.2020 Gazette 2020/21)**

(54) **SCALABLE MANUFACTURING WITH LASER INDUCED REFRACTIVE INDEX CHANGE**

SKALIERBARE HERSTELLUNG MIT LASERINDUZIERTER BRECHUNGSINDEXÄNDERUNG

FABRICATION ÉVOLUTIVE À CHANGEMENT D'INDICE DE RÉFRACTION INDUIT PAR LASER

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.11.2018 US 201862768387 P**

(43) Date of publication of application:
**25.08.2021 Bulletin 2021/34**

(73) Proprietor: **University of Rochester
Rochester, NY 14642 (US)**

(72) Inventors:
• **KNOX, Wayne H.**
**Rochester, New York 14612 (US)**
• **HUANG, Ruiting**
**Rochester, New York 14623 (US)**

(74) Representative: **Flügel Preissner Schober Seidel
Patentanwälte PartG mbB
Nymphenburger Straße 20
80335 München (DE)**

(56) References cited:
**WO-A1-2015/040338**  **US-A1- 2009 188 901**
**US-A1- 2010 228 345**

**Description**

Femtosecond micromachining has been utilized to create highly localized microscopic structures inside materials, including three-dimensional microfabrication within silica glasses, buried tubular waveguides written in bulk poly(methyl methacrylate), and channel waveguides or diffraction gratings formed in cross-linkable PMMA-based copolymers, owing to its unique characteristics, such as rapid and precise energy deposition into the materials, elimination of thermal diffusion to the surrounding area, structural modification within the focal volume, and etcetera. Over the last decade, this technique has been applied to alter the optical properties of ophthalmic materials, such as hydrogel-based contact lenses, intra-ocular lenses (IOL), and cornea tissues, by creating different refractive index shaping structures. Gandara-Montano *et al.* successfully wrote arbitrary Zernike polynomials in hydrogel-based contact lenses. A phase-wrapped lens was written directly inside an IOL to alter the hydrophilicity of targeted areas, and also high-quality gradient-index (GRIN) Fresnel lenses with wide power range from -3.0 to +1.5 diopters were written in plano contact lens materials. NIR/Blue femtosecond lasers have been used for noninvasive Intra-Tissue Refractive Index Shaping (IRIS) inside corneal tissue and even in the eyes of living cats.

There are many factors that can determine the effectiveness of a laser-induced refractive index change (LIRIC), including both material properties and laser exposure parameters. Material properties including chemical composition, water content, dopant type or dopant concentration have been studied so as to extend the limit of maximum achievable refractive index change.

The Raman spectrum signature arising from O-H stretching vibration of water centered at 3420 cm$^{-1}$ shows that larger phase change is associated with higher water content in the modified region and the lower refractive index of water compared to the studied hydrogels may account for the negative phase change induced in the laser-treated area. The function of water in the femtosecond micromachining process has been considered as increasing the heat-induced breakdown threshold due to the high heat capacity, slowing down the heat dissipation from the laser-treated area into the surrounding due to low heat diffusivity, or facilitating the photo-induced hydrolysis of polymeric materials in aqueous media.

As for the chemical composition needed for at least some femtosecond writings, there are two active components, a dopant and a quencher, to increase the energy deposited via nonlinear absorption and to transfer the energy absorbed for the chemical reaction to take place. Doped hydrogels have been reported to produce much larger RI change than undoped hydrogels because of enhanced nonlinearity absorption coefficient.

Apart from material properties, the induced RI change is also highly dependent on the system parameters and the effects of many system parameters, such as the scan speed, the laser average power, the numerical aperture (NA), the pulse width, the laser beam wavelength, and the number of layers written in the same area. Many of these parameters have been widely studied so as to optimize the femtosecond micromachining process. It has been reported that larger refractive index change can be induced in hydrogel polymers with a lower scan speed, a higher average power, and a shorter laser wavelength.

While progress has been made in the past studying factors that impact the effectiveness of the laser-induced refractive index change (LIRIC) in some materials, there remains room for improvement.

WO 2015/040338 A1 discloses a device for marking an ophthalmic lens and a laser marking process configured to produce permanent engravings on an ophthalmic lens by means of such a device. The device comprises a laser configured to produce permanent engravings on the lens and configured to emit a focused beam of pulsed ultraviolet laser radiation that comprises at least one radiation wavelength comprised between 200 nm and 300 nm, has a pulse length comprised between about 0.1 ns and about 5 ns, and has an energy per pulse comprised between about 5 $\mu$J and about 100 $\mu$J.

A laser material processing system is described in US 2009/0188901 A1. The system comprises a laser operable to emit a laser beam pulse; a shaper operable to shape the pulse; a hollow waveguide located in the path of the pulse to broaden the bandwidth of the pulse greater than about 20 nm; and a workpiece micro-machined by the pulse.

US 2010/0228345 A1 discloses a lens for placement in a human eye, such as intraocular lens. The lens has at least some of its optical properties formed with a laser. The laser forms modified loci in the lens when the modified loci have a different refractive index than the refractive index of the material before modification. Different patterns of modified loci can provide selected dioptic power, toric adjustment, and/or aspheric adjustment provided.

SUMMARY

This patent describes methods for optimizing manufacturing processes utilizing laser-induced refractive index changes, such as changes induced in ophthalmic materials by femtosecond laser micromachining. Applications of the techniques described in this patent include LIRIC customization of contact lenses, intra-ocular lenses (ex-vivo), and other ophthalmic materials.

This patent also describes a photochemical model that we have developed for LIRIC that can be used to design commercial scale LIRIC manufacturing systems and methods. We have tested our photochemical model in two-photon mode for blue writing in hydrogels and four-photon mode with 1035 nm writing in hydrogels. As just one example, our model predicts very fast writing with green 517 nm up to 3.7 m/sec demonstrated with 500 mw at 10 MHz. More particularly, the invention proposes a method of designing a laser writing system for modifying a plurality of ophthalmic devices in accordance with claim 1.

One example of a method for designing a laser writing system for modifying a plurality of ophthalmic devices includes the steps of: (a) determining at least one material characteristic of the ophthalmic devices, determined over a range of laser writing system parameters; (b) determining at least one design characteristic of the ophthalmic device; and (c) using at least the determined material and design characteristics, configuring at least one system parameter of the laser writing system to optimize throughput of the laser writing system, the laser writing system including: (i) a laser configured to generate a laser beam, (ii) a splitter configured to split the laser beam into a plurality of outputs, and (iii) a plurality of writing heads, each writing head configured to direct at least one of the outputs to an ophthalmic device to write one or more localized refractive index modifications into the ophthalmic device.

In some instances, the at least one material characteristic includes a damage threshold for an induced single layer optical phase shift of the ophthalmic device; and the at least one design characteristic includes a maximum phase shift designed for the ophthalmic device.

In some instances, the at least one material characteristic includes at least a first damage threshold for an induced single layer optical phase shift of the ophthalmic device at a first laser repetition rate, and a second damage threshold for an induced single layer optical phase shift of the ophthalmic device at a second laser repetition rate.

In some instances, the range of laser writing system parameters used for determining the at least one material characteristic includes at least one of a power range, a scan speed range, a laser repetition rate range, and a range of focusing lens numerical aperture.

In some instances, the range of laser writing system parameters used for determining the at least one material characteristic includes at least two of the power range, the scan speed range, the laser repetition rate range, and the focusing lens numerical aperture.

In some instances, the determined material characteristics also include at least one fitting parameter of a quantitative model relating an induced phase shift in the ophthalmic device to a configuration of the laser writing system.

In some instances, the determined material characteristics also include a multiphoton order of the quantitative model.

In some instances, the quantitative model is a two photon regime model such as:

$$\Delta\phi(P,v,S,NA) := \beta \cdot \left( \frac{P^2 \cdot NA}{v \cdot \lambda^3 \cdot S \cdot \tau} \right)$$

in which $\Delta\phi$ is a single layer written phase shift, $P$ is an average power, $NA$ is a numerical aperture, v is a laser repitition rate, $\lambda$ is a laser wavelength, $S$ is a scan speed, $\tau$ is a pulse duration, and $\beta$ is the fitting parameter.

In some instances, the quantitative model is a three photon regime model such as:

$$\Delta\phi := \frac{\gamma \cdot P^3 \cdot NA^3}{v^3 \cdot \tau^2 \cdot \lambda^5 \cdot S}$$

in which $\Delta\phi$ is a single layer written phase shift, $P$ is an average power, $NA$ is a numerical aperture, v is a laser repitition rate, $\lambda$ is a laser wavelength, $S$ is a scan speed, $\tau$ is a pulse duration, and $\gamma$ is the fitting parameter.

In some instances, the quantitative model is a four photon regime model such as:

$$\Delta\phi = \gamma \cdot \frac{P^4 \cdot NA^5}{v^3 \cdot \tau^3 \cdot S \cdot \lambda^7}$$

in which $\Delta\phi$ is a written single layer phase shift, $P$ is an average power, $NA$ is a numerical aperture, v is a laser repitition rate, $\lambda$ is a laser wavelength, $S$ is a scan speed, $\tau$ is a pulse duration, and $\gamma$ is the fitting parameter.

In some instances, the quantitative model also includes a saturation factor.

In some instances, the quantitative model is an Nth photon regime model such as:

$$\Delta\phi := \frac{\gamma \cdot P^N \cdot NA^{2 \cdot N - 3}}{v^{N-1} \cdot \tau^{N-1} \cdot \lambda^{2 \cdot N - 1} \cdot S}$$

in which $\Delta\phi$ is a single layer written phase shift, $P$ is an average power, $NA$ is a numerical aperture, v is a laser repitition rate, $\lambda$ is a laser wavelength, $S$ is a scan speed, $\tau$ is a pulse duration, and $\gamma$ is the fitting parameter.

In some instances, the multiphoton order of the quantitative model is a two photon regime, a three photon regime, or a four photon regime.

In some instances, the quantitative model comprises a sum of a plurality of multi-photon regime models.

In some instances, the quantitative model is:

$$\Delta\Phi := \beta \cdot \frac{P^2 \cdot NA}{v \cdot \tau \cdot \lambda^3 \cdot S} + \gamma \cdot \frac{P^3 \cdot NA^3}{v^2 \cdot \tau^2 \cdot \lambda^5 \cdot S} + \delta \cdot \frac{P^4 \cdot NA^5}{v^3 \cdot \tau^3 \cdot \lambda^7 \cdot S}$$

in which $\Delta\phi$ is a single layer written phase shift, $P$ is an average power, $NA$ is a numerical aperture, v is a laser repitition rate, $\lambda$ is a laser wavelength, $S$ is a scan speed, $\tau$ is a pulse duration, $\beta$ is a second order fitting parameter, $\gamma$ is a third order fitting parameter, and $\delta$ is a fourth order fitting parameter.

In some instances, optimizing throughput of the laser writing system includes determining an optimal number of writing layers for modifying the ophthalmic devices in combination with an optimal number of writing heads of the laser writing system for optimizing throughput of the laser writing system.

In some instances, optimizing throughput of the laser writing system includes determining at least one of an optimal laser repetition rate, laser wavelength, laser pulsewidth, and scan speed for optimizing throughput of the laser writing system.

In some instances, determining the at least one material characteristic of the ophthalmic devices includes determining the at least one material characteristic over a first range of laser writing system parameters and a second range of laser writing system parameters, the first and second ranges including at least one different laser repetition rate parameter, laser wavelength parameter, laser pulsewidth parameter, and scan speed parameter.

In some instances, the design method also includes determining a line spacing characteristic of the ophthalmic device.

One example of a laser writing system for modifying a plurality of ophthalmic devices, includes: (a) a laser configured to generate a laser beam, (b)a splitter configured to split the laser beam into a plurality of outputs, and (c) a plurality of writing heads, each writing head configured to direct at least one of the outputs to an ophthalmic device to write one or more localized refractive index modifications into the ophthalmic device; in which the laser writing system is configured in accordance with the method described above.

In some instances, the laser is configured to generate a pulsed laser beam having a pulsewidth less than 350 femtoseconds and a repetition rate between 1 and 60 MHz.

In some instances, the laser is configured to generate a pulsed laser beam having a wavelength in the range of 340 nm to 1100 nm.

In some instances, the laser is configured to generate a pulsed laser beam having a wavelength in the range of 515 nm to 520 nm, or 1030 nm to 1040 nm, or 400 nm to 410 nm, or 795 nm to 805 nm.

In some instances, the splitter is configured to split the laser beam into 2 to 64 outputs.

In some instances, the ophthalmic devices are contact lenses or intraocular lenses, or hydrogel corneal implants.

One example of a method of using a laser writing system for writing localized refractive index changes into ophthalmic devices includes providing a laser writing system as described above configured in accordance with a method described above, and (a) generating a pulsed laser beam in the laser writing system; (b) splitting the pulsed laser beam into a plurality of outputs, at least some of the outputs each associated with a writing head in the laser writing system; and (c) at each writing head, scanning the pulsed laser beam relative to an ophthalmic device to write one or more refractive index changes into the ophthalmic device.

One example of a method of using a particular laser writing system for writing localized refractive index changes into particular ophthalmic devices includes: (a) generating a pulsed laser beam having a wavelength in the range of 1030 nm to 1040 nm, a pulsewidth less than 350 femtoseconds, and a repetition rate less than 20 MHz; (b) splitting the pulsed laser beam into a plurality of outputs, at least some of the outputs each associated with a writing head; (c) at each writing head, scanning the pulsed laser beam relative to an ophthalmic device to write one or more refractive index changes into the ophthalmic device, wherein the laser writing system is configured such that it is capable of inducing a single writing layer phase shift of at least 0.3 waves at an average power at the writing head of less than 1500 mW and a scan speed greater than 100 mm/s.

In some instances, the ophthalmic device is a hydrogel material having a four photon absorption parameter in the range of 1e-59 m$^6$·waves/(W$^4$·s) to 3e-59 m$^6$·waves/(W$^4$·s) in the equation:

$$\Delta\phi = \gamma \cdot \frac{P^4 \cdot NA^5}{\upsilon^3 \cdot \tau^3 \cdot S \cdot \lambda^7}$$

in which $\Delta\phi$ is a written single layer phase shift, $P$ is an average power of the pulsed laser beam at the writing head, $NA$ is a numerical aperture of the writing head, v is a laser repitition rate, $\lambda$ is a laser wavelength, $S$ is a scan speed, $\tau$ is a laser pulse duration, and $\gamma$ is the four photon absorption parameter.

BRIEF DESCRIPTION OF THE DRAWINGS

Figure 1 illustrates a femtosecond writing system. The Yb doped fiber laser is able to deliver femtosecond pulses at a series of adjustable repetition rates and with maximum output power up to 30 W.

Figures 2(a)-(b) are DIC images of the grating lines written at an average power of ~500 mW, at scan speeds of 5 mm/s, 50 mm/s and 100 mm/s (from top to bottom), and at repetition rates of 15 MHz (a) and 60 MHz (b), respectively.

Figures 2(c)-(d) are DIC images of the grating lines written at average powers of ~500 mW, ~1000 mW and ~1500 mW (from top to bottom), at a scan speed of 100 mm/s, and at repetition rates of 15 MHz (c) and 60 MHz (d), respectively.

Figure 3(a) is a DIC image of four phase bars written in a J+J contact lens at two different powers, 1075 mW (top two) and 1105 mW (bottom two).

Figure 3(b) is a schematic diagram showing two different scanning methods - raster scanning (left) and rectangular loop scanning (right).

Figure 3(c) is an interferogram of the two phase bars written at a power of 1105 mW, a scan speed of 200 mm/s, and a repetition rate of 15 MHz.

Figure 3(d) is a phase map showing phase difference between the laser-treated area and the surroundings.

Figures 4(a)-(d) illustrate quantitative results showing the phase change magnitude measured at 543 nm for the phase bars written in a J+J Acuvue contact lens as a function of power (Fig. 4(a)) and as a function of single pulse energy (Fig. 4(b)); phase bars written in a Contaflex GM Advance 58 sample as a function of power (Fig. 4(c)) and as a function of single pulse energy (Fig. 4(d)) at different repetition rates, 5 MHz, 10 MHz, 15 MHz and 60 MHz.

Figures 5(a) and 5(b) are plots of logarithm of the induced phase change at 543 nm versus logarithm of the average power in the focal volume at 5 MHz, 10 MHz, 15 MHz and 60 MHz for the Contaflex sample (a) and the J+J contact lens (b) for the purpose of the determination of the order of multiphoton absorption process.

Figure 6 illustrates quantitative results showing the phase change magnitude measured at 543 nm written in a Contamac 58 sample as a function of power.

Figure 7 is a plot of logarithm of the induced phase change at 543 nm versus logarithm of the average power in the focal volume for the Contamac 58 sample.

Figure 8 schematically illustrates one example of LIRIC multi-head writing system.

Figure 9 schematically illustrates one example of a high power splitter arrangement usable with the LIRIC multi-head writing system of Figure 8.

Figure 10 schematically illustrates one example of a scanning and delivery subsystem usable with the LIRIC multi-head writing system of Figure 8.

Figure 11 illustrates a pulse energy range in which a refractive index change occurs below a threshold in which gross material damage or scattering effects would occur.

Figure 12 illustrates a scan speed range in which a refractive index change occurs above a threshold in which gross material damage or scattering effects would occur.

Figure 13 illustrates examples of LIRIC lenses created in plano hydrogel.

Figure 14 illustrates an example of a Fresnel-type phase-wrapped optical lens written in a thin layer.

Figure 15 is data plotting phase change as a function of power and speed in Johnson & Johnson Acuvue2 contacts in response to 100 fs laser pulses at a repetition rate of 80 MHz and 800 nm, for speeds of (a) 3 mm/s, (b) 5 mm/s, (c) 10 mm/s, (d) 15 mm/s, (e) 20 mm/s, (f) 30 mm/s, and (g) 40 mm/s.

Figure 16 is a three-dimensional visualization of the data in Figure 15.

DETAILED DESCRIPTION

The following detailed description sets out examples of LIRIC writing systems and methods for ophthalmic materials, as well as techniques using our photochemical models for improving LIRIC manufacturing systems and methods. The specific examples described below are for illustrative purposes only, and are not intended to limit the scope of our inventions. Changes could be made to the systems, methods, and techniques described below without departing from the scope or spirit of our inventions.

### Laser Pulse Repetition Rate

In some of the examples described in this patent, in order to enhance the feasibility and practicability of the femtosecond micromachining in vision correction, we rely on the laser pulse repetition rate to reduce the average power and to enhance the scan speed needed for inducing detectable refractive index (RI) change inside ophthalmic materials. There are several research groups that have reported the effect of the repetition rate on the microstructure formation inside fused silica and metals with the range of Hz and KHz. Most of the studies are focused on the effect of repetition rate on ablation efficiency, laser drilling of metals, defects formation, or surface texture of the laser induced microstructures. A comparison between kilohertz and megahertz laser systems made by Reichman *et al.* showed that megahertz repetition rate modification resulted in better quality waveguides with a greater refractive index increase than kilohertz repetition rate modification. However, to our best knowledge, no studies so far have illustrated the effect of repetition rate on the RI change induced by femtosecond micromachining in hydrogel-based polymers.

The typical repetition rate currently used in femtosecond micromachining hydrogel polymers is larger than 80 MHz. However, prior to our investigation, it has not been investigated whether the high repetition rate (> 80 MHz) is the optimal repetition rate for writing ophthalmic materials without inflicting any optical damage. Given the same single pulse energy, the accumulated temperature rise induced by a high-repetition-rate pulse train is higher than a low-repetition-rate pulse train, indicating that the high repetition rate of the pulse train should be able to induce larger refractive index change if we assume a pyrolytic degradation of polymers. However, we have considered the possibility that the laser-induced RI change results from a combination of both thermal-induced decomposition process and direct photochemical changes made by the nonlinear multiphoton absorption process. Although the accumulated thermal effect might be more significant in high repetition rate region, the photon energy that can be absorbed by the material is diminished due to a smaller single pulse energy for the same amount of average power; on the other hand, the low-repetition-rate pulses may easily reach the optical breakdown threshold and cause gross damage at smaller average power due to a larger single pulse energy. Therefore, we investigated the effect of repetition rate on both the induced RI change and the optical damage threshold. As part of our investigation, we discovered that the optimal repetition rate can be taken advantage of to reduce the amount of power required and speed up the femtosecond machining process.

In the following sub-sections, we present both the qualitative and quantitative experimental results showing how the repetition rate affects the magnitude of the induced phase change, which is related to RI change via the following equation,

$$\Delta\phi = \frac{\Delta n \cdot L}{\lambda} \qquad\qquad (1)$$

where $\Delta\phi$ is the magnitude of the induced phase change in number of waves and measured at a specific reference wavelength (e.g. in this example at $\lambda$=543 nm), $\Delta n$ is the induced average RI change, and $L$ is the longitudinal thickness of the laser induced region. Grating lines were written in hydrogel-based contact lenses at two different repetition rates, 15 MHz and 60 MHz, to show that the great discrepancy between low repetition rate modification and high repetition rate modification can be easily visualized from the differential interference contrast (DIC) images. More systematic quantitative results were obtained by fabricating continuous phase shifting bars at four different repetition rates, 5 MHz, 10 MHz, 15 MHz and 60 MHz. A calibration function is derived from a photochemical model by associating the induced phase change with the molecular density changes due to the multiphoton absorption process and a pulse overlapping effect. The coeffiecients in the calibration function are fitted by the least square method. Single pulse energy damage threshold as a function of repetition rate was also investigated so as to determine the maximum achievable phase change just below the material damage threshold and to obtain the dynamic range for the writing process.

### 1. Experimental Setup

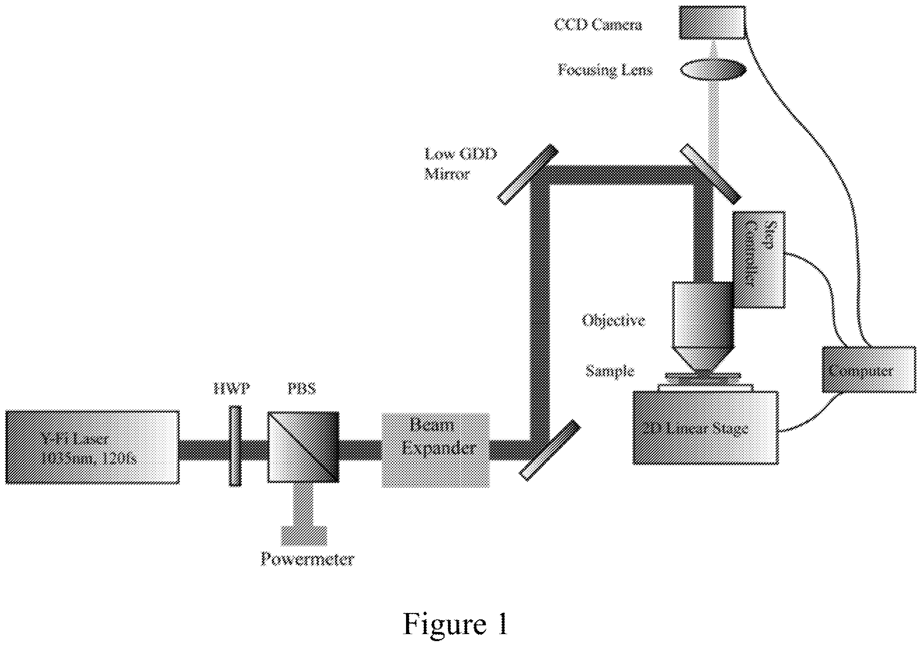

The system configuration used for our experiment is depicted in Figure 1. The light source used for the writing process is a KM Lab Y-Fi (Ytterbium fiber) laser, which delivers femtosecond laser pulses with a pulse duration of ~120 fs, a central wavelength at 1035±5 nm. The pulse duration is measured by a commercially available autocorrelator (Newport, PSCOUT2-NIR-PMT) and can be adjusted by an internal grating pair working as a pulse compressor. This powerful Y-Fi laser contains an all-normal-dispersion (ANDi) Yb doped fiber oscillator, which enables tunable repetition rates in the range from 1 MHz to 60 MHz via the usage of a pulse picker. The pulse repetition rate from the oscillator is fixed at 60 MHz, and a pulse picker extracts certain pulses from the fast pulse train with an identical temporal separation, resulting in a different repetition rate from the oscillator. The available repetition rates are 60/$N$ MHz, where $N$ is an integer number from 4 to 60. An amplifier is employed after the pulse picker to amplify the average power up to 30 W, corresponding to a maximum pulse energy of 0.5 $\mu$J at 60 MHz. A HWP and a PBS are placed after the Y-Fi laser to split the incident linearly polarized beam into two beams with orthogonal polarization and controllable power. The split ratio of the HWP and PBS unit was set to be 90:10, where 90% of the incoming beam transmits through the PBS and 10% of the beam is reflected onto a power meter for monitoring the power used in the writing process. A beam expander, consisting of two positive lenses, is placed after the HWP and PBS unit to amplify the beam size so that the effective numerical aperture (NA) for writing the samples is 0.47, and the corresponding diffraction-limited spot size is 2.75 $\mu$m. Three low GDD mirrors (Thorlabs, UM10-45B) are inserted in the system to fold the optical beam path and then the beam is sent through a microscope objective (Olympus, LCPLN50XIR) to form a diffraction-limited spot inside a sample. The microscope objective is attached to a motorized vertical stage (Newport, GTS30V) which provides Z axis step control. A sample is sandwiched between a microscope glass slide and a cover slip and soaked with solution to maintain hydrated. The sample is then mounted on a 2D linear translation stage (Aerotech, PRO115LM) which allows XY axial scanning with a speed up to 3 m/s. A back reflection monitor consisting of a focusing lens and a CCD camera is used to locate the writing depth which is was usually set to be in the middle of the polymer sample.

### 2. Experimental Results

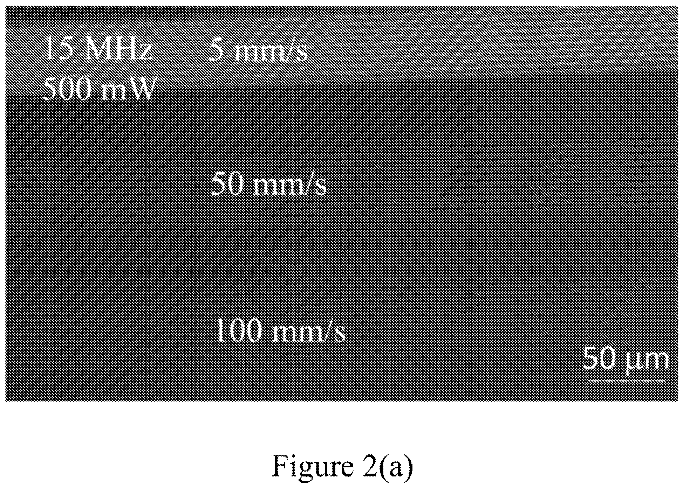

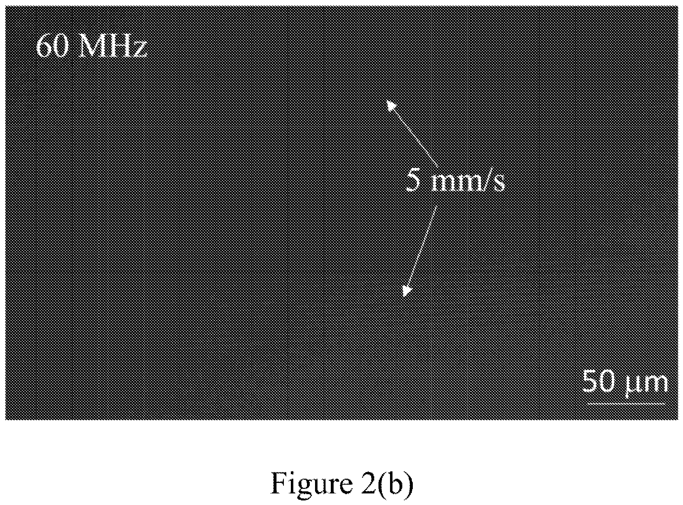

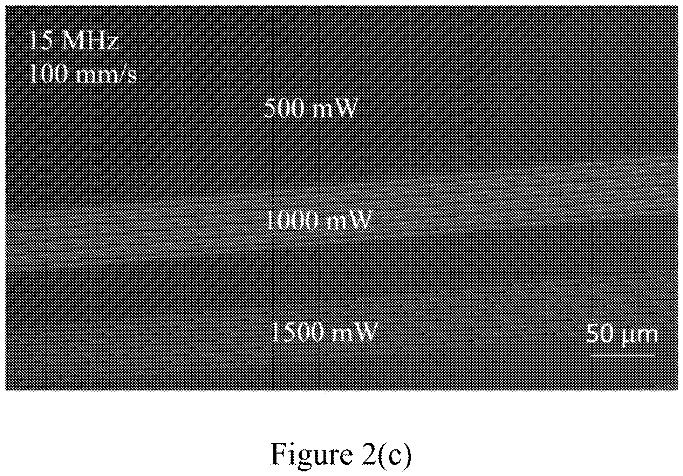

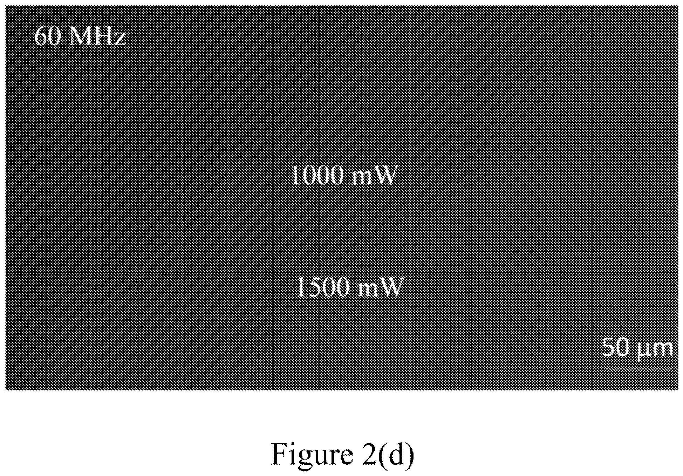

In order to illustrate the effect of repetition rates on the induced phase change, we conducted both qualitative and quantitative experiments at different repetition rates, with different powers and different scan speeds. Samples used in our qualitative experiments are hydrogel-based contact lenses (Acuvue2, Johnson & Johnson) made of a soft hydrophilic material known as "etafilcon A", a copolymer of 2-hydroxyethyl methacrylate and methacrylic acid cross-linked with 1, 1, 1-trimethylol propane trimethacrylate and ethylene glycol dimethacrylate. Periodic grating structures were fabricated ~50 $\mu$m below the top surface of a contact lens using a raster scanning method. Figures 2(a) and (b) show the DIC images of the gratings written with a power of ~500 mW, at three different scan speeds, 5 mm/s, 50 mm/s and 100 mm/s and at two different repetition rates of 15 MHz and 60 MHz. Each grating consists of 10 grating lines with a line spacing of 5 $\mu$m, resulting in a total width of 50 $\mu$m. In order to maintain a constant velocity inside the sample, the length of each grating is set to be 20 mm, which is ensured to be long enough to compensate for the acceleration and deceleration travel distance of the stage. Given the same average power and the same repetition rate, the grating lines became fainter as the scan speed was increased, indicating that a larger RI change can be induced using a smaller scan speed. Distinct difference can be perceived between the DIC image obtained at 15 MHz (Fig. 2(a)) and 60 MHz (Fig. 2(b)). Grating lines micro-machined at 15 MHz can be seen at all the three scan speeds while the grating lines are difficult to be detected at 60 MHz even at the lowest scan speed. Results obtained at 100 mm/s using three different powers, 500 mW, 1000 mW and 1500 mW and two different repetition rates are shown in Fig. 2(c) and (d), respectively. Similar results were obtained from the power scaling experiments as from the scan speed scaling experiments. Grating lines became brighter as the power was increased until the optical breakdown threshold was reached while the repetition rate and the scan speed were kept the same. The optical damage was indicated by the formation of dark carbon spots or material distortion with melting traces and porosities highly localized along the grating lines. The results obtained at the same repetition rate are consistent with the conclusions drawn in Ding *et al.* 's paper, implying that larger RI change can be achieved by increasing the average power and lowering the scan speed. However, comparing the DIC images at two different repetition rates while keeping other irradiation parameters the same leads to several new findings. As shown by the bright central grating lines written at 15 MHz (Fig. 2(c)) and the fairly faint grating lines at 60 MHz (Fig. 2(d)), the usage of a lower repetition rate pulse train can result in a much larger refractive index change at the same average power. Lower-repetition-rate pulses also cause optical damage at a smaller average power, as indicated in the images that for a given average power at 1500 mW, carbonized grating lines could be observed at 15 MHz while minor modification was induced at 60 MHz. The noteworthy difference between 15 MHz and 60 MHz can be explained from the single pulse energy point of view. Single pulse energy as a product of the average power and pulse interval is 4 times larger at 15 MHz than at 60 MHz for the same average power, meaning more energy per pulse can be absorbed by the material through multiphoton absorption at a lower repetition rate to induce more significant amount of RI change.

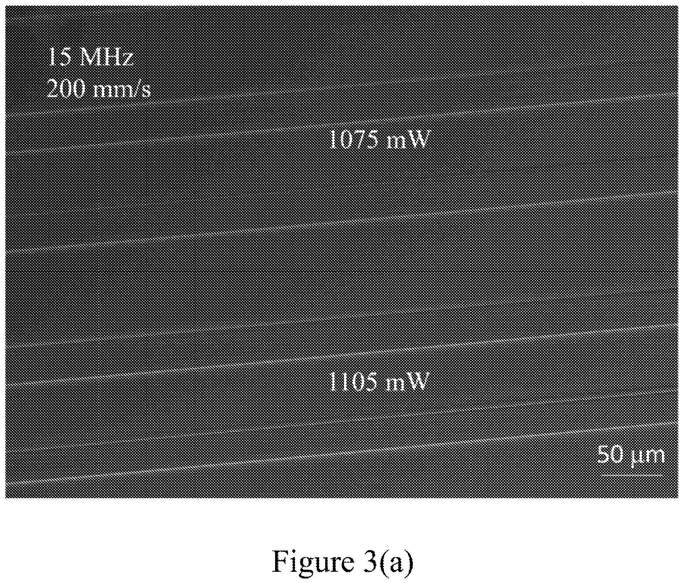

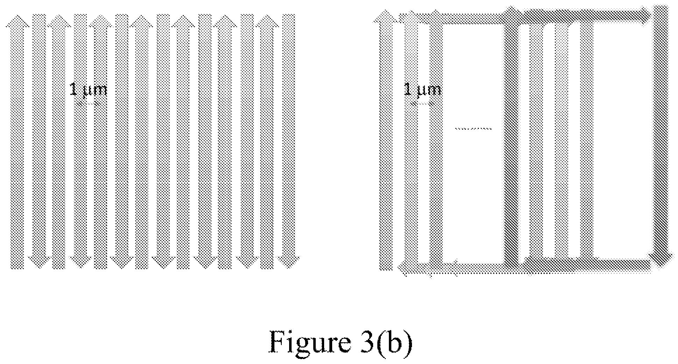

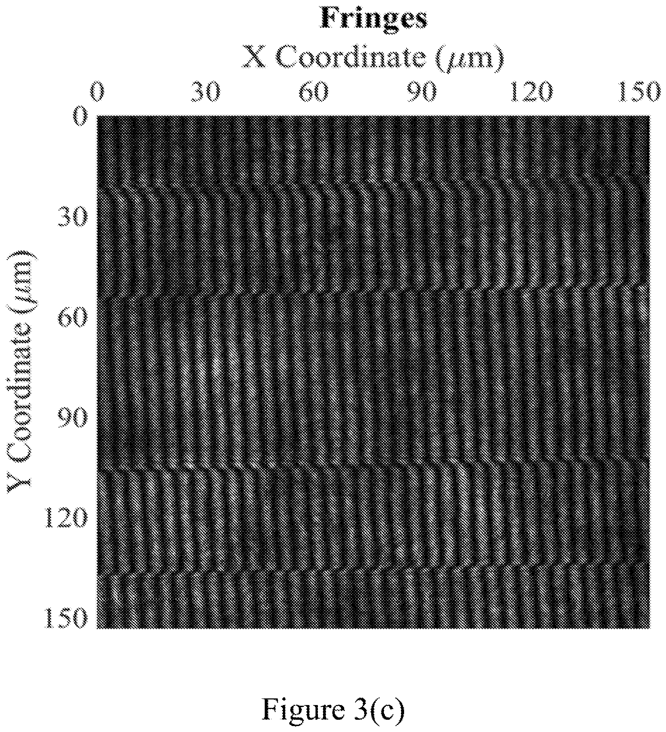

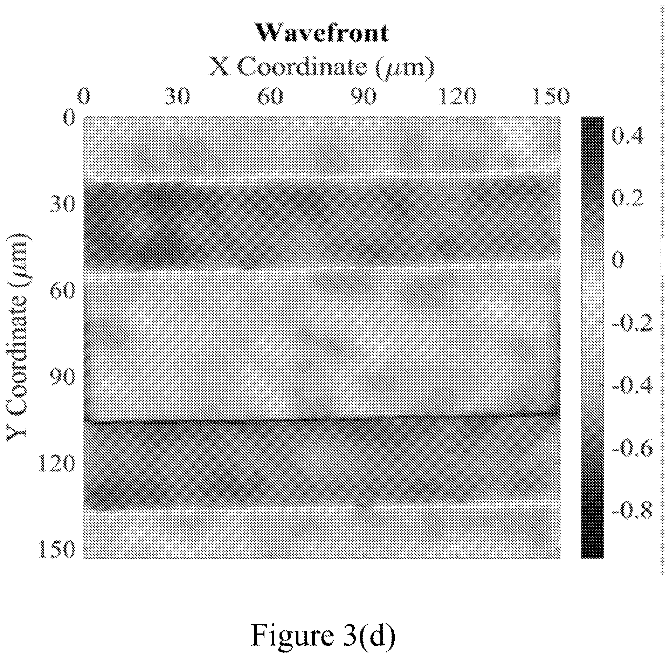

More systematic experiments were performed to generate quantitative results. We inscribed phase bars instead of grating lines inside a Acuvue J+J contact lens with +4 diopters and a base curvature of 8.7 mm. The travel distance of the 2D linear stage is still set to be 20 mm so as to keep the scan speed constant at 200 mm/s inside the sample. Each phase bar is made up of 30 grating lines separated by 1 $\mu$m, which is smaller than the focused spot size, thus creating a continuous phase carpet of dimensions 30 $\mu$m by 20 mm. As shown in Fig. 3(a), there are 4 phase bars written at two different exposure conditions. The top two phase bars were fabricated at 1075 mW while the bottom ones at 1105 mW. The difference between two writing conditions (as long as no damage occurs) cannot be detected directly from the DIC image but can be well spotted by measuring the phase change induced inside the written area with respect to the surroundings under a home built two-wavelength Mach-Zehnder interferometer (MZI). The scanning method used in the experiments is a rectangular loop scanning as shown in Fig. 3(b) rather than a raster scanning, which allows us to write side by side two same phase bars with a separation of 100 $\mu$m. Additionally, the rectangular loop scanning minimizes the spatial thermal effect induced by two adjacent grating lines since the separation between two adjacent grating lines is 100 times larger than that in the raster scanning. The amount of phase change measured with a reference light at 543 nm is extracted and unwrapped via the Goldstein's branch cut unwrapping algorithm and Fourier-transform method of fringe pattern analysis. The interferogram showing two phase bars written at the same irradiation parameters (used for creating the two bottom phase bars in Fig. 3(a)) and the corresponding retrieved phase map are displayed in Fig. 3(c) and (d), respectively. Obvious phase shift can be observed from the interferogram and phase change inside the written region differs from the pristine region with sharp discontinuity at the edges. The average phase change was calculated to be -0.54 waves and the variation inside one phase bar was less than 0.05 waves. The difference of the induced phase change between the top phase bar and the bottom one in Fig.3 (d) is roughly 0.02 waves, indicating that consistent and stable phase change can be achieved by the femtosecond micromachining process. The longitudinal thickness of each scanning line is estimated to be around 8 $\mu$m by taking a cross section image of one grating structure consisting of 10 scanning lines and therefore the corresponding RI change to a phase change of -0.54 waves at 543 nm is calculated to be -0.037 from Eq. (1).

3. Quantitative Photochemical Model of Phase Changes

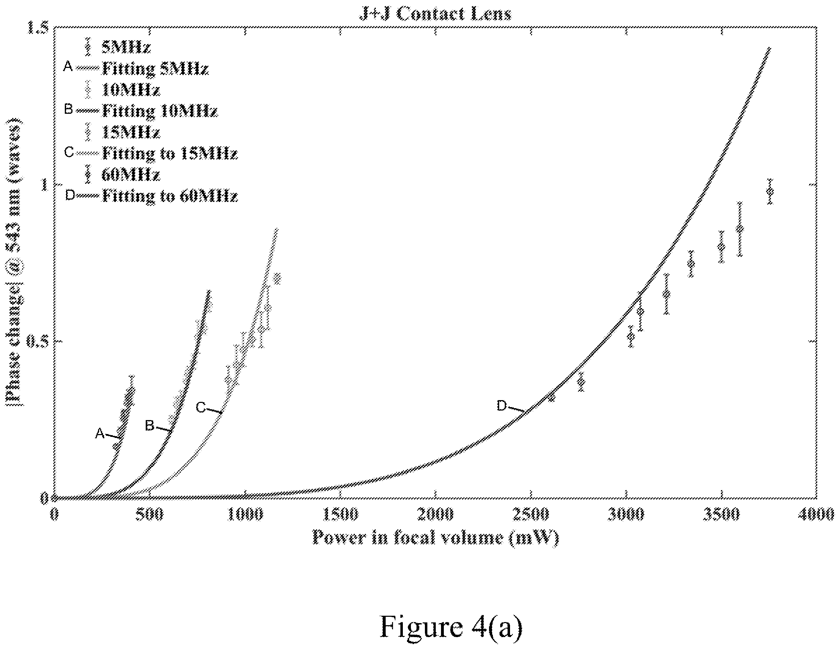

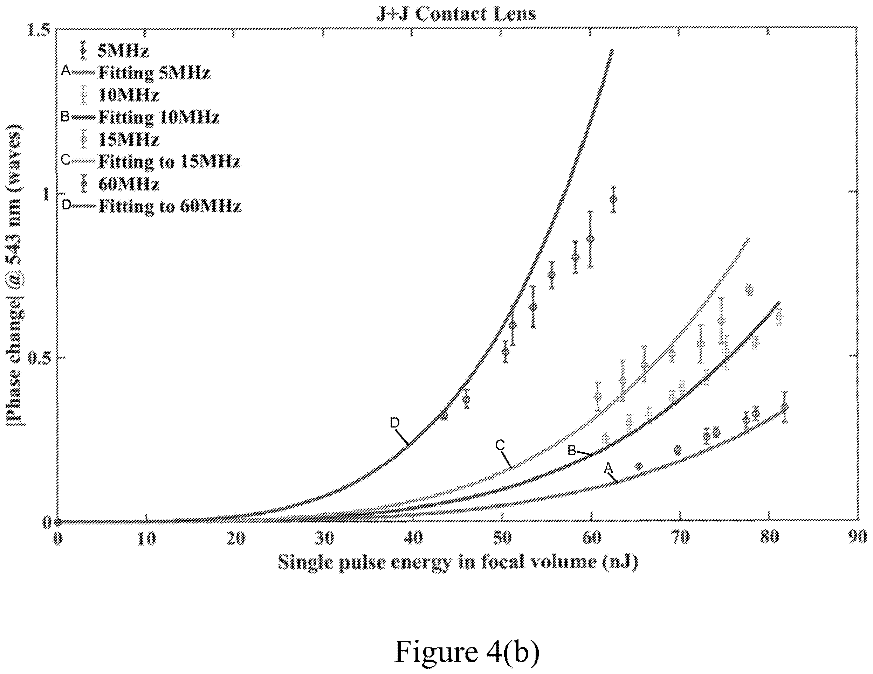

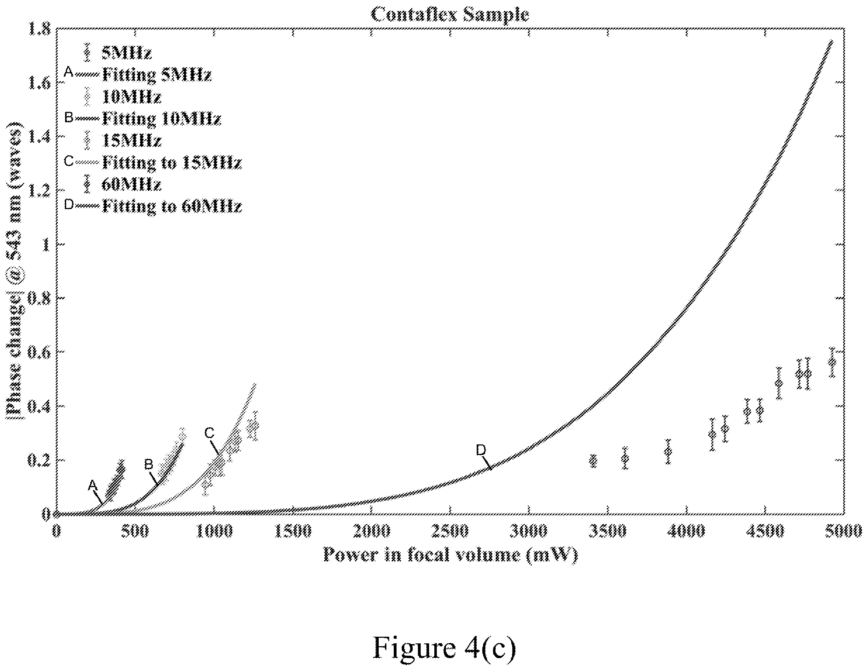

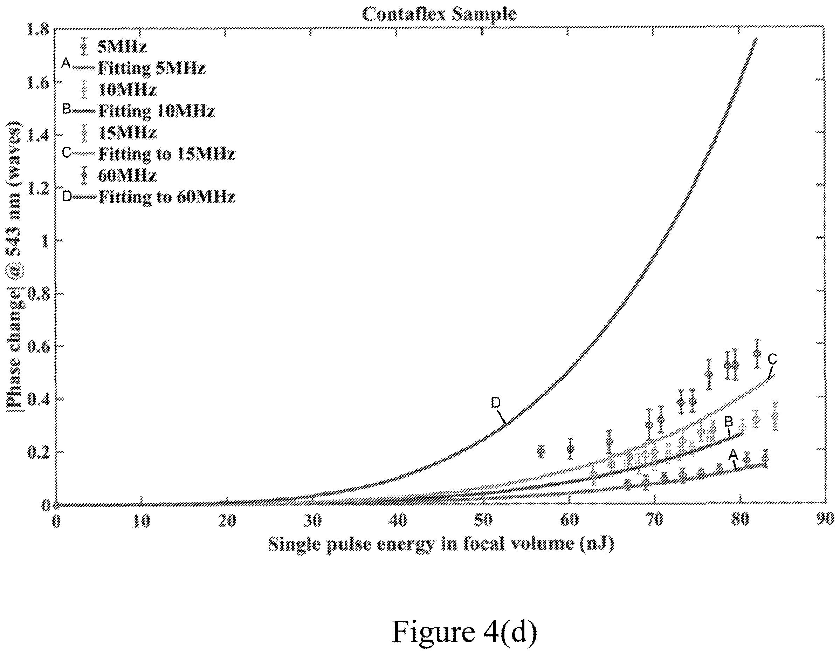

To map out the induced phase change at different repetition rates, the power tested started from a small value at which a small phase change can be measured by the MZI and ended at a value where the sample damage occurs. The scanning pattern was the rectangular loop scanning and the writing speed was fixed at 200 mm/s. Two materials were laser-treated for collecting quantitative data at four different repetition rates, 5 MHz, 10 MHz, 15 MHz and 60 MHz. One of the materials is an Acuvue J+J Contact lens as described in the previous section and the other one is a plano hydrogel sample named Contaflex GM Advance 58 (Contamac Inc.), which is made of "Acofilcon A", a synonym for "2-Butenedioic acid (2Z)-, di-2-propenyl ester, polymer with 2,3-Dihydroxypropyl 2-methyl-2-propenoate, 1-Ethenyl-2-pyrrolidinone, 2-Hydroxyethyl 2-methyl-2-propenoate and Methyl 2-methyl-2-propenoate". The magnitude of the measured phase change at four repetition rates as a function of average power and single pulse energy is shown respectively in Fig. 4(a) and 4(b) for the J+J contact lens as well as in Fig. 4(c) and 4(d) for the Contaflex GM Advance 58 sample. Each experimental data was obtained by averaging the phase change values from 8 to 12 phase bars that were written with the same laser irradiation parameters. The variance assigned to each individual data shows the standard deviation obtained from these rectangles.

The fitting curves are derived from a photochemical model involving multiphoton absorption process and overwriting factor. RI change is assumed to be induced by the density change of polymer matrix in the excitation volume through multiphoton absorption. In our specific case where near infrared light at 1035 nm was used, a four photon absorption process was expected to occur during the writing process due to a strong UV linear absorption cut off wavelength around 300 nm. Then the excited molecular density change D can be expressed in the following form assuming a small absorption limit for simplicity,

$$D = \varepsilon \cdot (\frac{E}{VOL}) \cdot (\beta \cdot I_{peak}^{3} \cdot L) \qquad (2)$$

where $\varepsilon$ is a material constant for relating the deposited energy to the molecule density change, $E$ is the single pulse energy absorbed by the material, $VOL$ is the light-matter interaction volume, $\beta$ is the fourth photon absorption coefficient, $I_{Peak}$ is the peak pulse intensity and $L$ is the longitudinal thickness of the interaction region. Apart from the four photon absorption process, accumulation effect originating from pulse overlapping should also be considered. The overwriting of spots can be accounted for as the spot size divided by the spacing between the spots by assuming a uniform ('top-hat') beam profile, denoted as for simplicity,

$$N = \frac{\omega \cdot v}{S} \qquad (3)$$

where $\omega$ is the diffraction limited laser spot size inside the material, $v$ is the pulse repetition rate and $S$ is the scan speed. Eq. (3) represents the number of pulses per spot and the detailed illustration about the intersection of displaced laser pulses can be found in other research work. Therefore, the number of pulses per spot can be calculated based on Eq. (3) to be ~18, ~35, ~53 and ~210 at 5 MHz, 10 MHz, 15 MHz and 60 MHz, respectively.

Following Eq. (2) and Eq. (3), the overall excited molecule density change can be expressed as the product of the molecule density change caused by a single pulse times the overlapping effect N. After mathematica manipulation and expressing the single pulse energy in terms of average power and repetition rate, we are able to propose the final result of the phase change equation,

$$\Delta\phi = \gamma \cdot \frac{P^4 \cdot NA^5}{v^3 \cdot \tau^3 \cdot S \cdot \lambda^7} \qquad (4)$$

where $P$ is the average power, $NA$ is the numerical aperture, $\tau$ is the pulse duration. Parameter $\gamma$ is a constant which contains the fourth photon absorption coefficient and associates the absorbed energy with the phase change. It can be determined by using a least square fitting method. Results show the fitting curve matches the experimental results well for the three low repetition rates but there is a deviation at 60 MHz between the experiemtnal data and the fitting curve. The fitting parameter is found to be $\gamma$ = 2.82e-59 m$^6$·waves/(W$^4$·s) for J+J contact lenses and $\gamma$ = 1.33e-59 m$^6$·waves/(W$^4$·s) for Contaflex samples. The results may suggest that the fourth photon absorption rate or the ability of transferring the absorbed energy to induce RI change of Contaflex samples is smaller than that of J+J contact lenses. Therefore, it may be considered to add dopants into the Contaflex samples to increase absorption cross section or add quenchers to enhance the energy conversion efficiency.

Several conclusions can be drawn from Fig. 4. As shown in Fig. 4(a) and 4(c), the magnitude of the phase change measured at 543 nm rises as the power increases for a given repetition rate for both materials and lower repetition rates can induce the same amount of phase change at a much smaller power, which are consistent with the qualitative results shown in Fig. 2. Lower repetition rate, on the other hand, causes sample damage at a smaller power for both materials; however, the damage threshold in terms of the single pulse energy behaves differently for two materials. As shown in Fig. 4(b), the single pulse energy needed for initiating the optical breakdown in a J+J contact lens is ~82 nJ at 5 MHz, ~81 nJ at 10 MHz, ~77 nJ at 15 MHz and ~63 nJ at 60 MHz. Therefore, the experimental results indicate that the sample damage threshold of a J+J contact lens decreases as the repetition rate increases or equivalently the number of laser shots at the same spot increases. However, Fig. 4(d) shows that the single pulse energy damage threshold of a Contaflex sample is ~83 nJ at 5 MHz, ~82 nJ at 10 MHz, 84 nJ at 15 MHz and 82 nJ at 60 MHz, indicating a damage threshold independent of repetition rate. We assume the relationship between the optical breakdown threshold and the number of pulses per spot can be described by the incubation effect. Given the same single pulse energy, the magnitude of the phase change increases as the repetition rate rises, which could be attributed to the accumulation effect arising from the overlapping of two adjacent pulses. A higher-repetition-rate pulse train is able to produce a larger number of laser shots on the same spot, meaning more pulse energy could be absorbed by the material. Therefore, it is reasonable to conclude that the maximum achievable phase change just below the damage threshold also increases as the repetition rate increases by taking into account that the change in single pulse energy damage threshold is modest for the repetition rates from 5 MHz through 60 MHz. As shown in Fig. 4(b) and 4(d), the maximum ahievable phase change for the J+J contact lens is ~0.38 waves at 5 MHz, ~0.6 waves at 10 MHz, ~0.7 waves at 15 MHz and ~1 wave at 60 MHz and for the Contaflex sample, ~0.17 waves at 5 MHz, ~0.26 waves at 10 MHz, ~0.33 waves at 15 MHz and 0.56 waves at 60 MHz. The increasing pulse overlapping effect at higher repetition rates and the roughly same single pulse energy threshold at different repetition rates together contribute to the growth of the maximum achievable phase change just below the damage threshold.

4. Empirical Model of Phase Changes

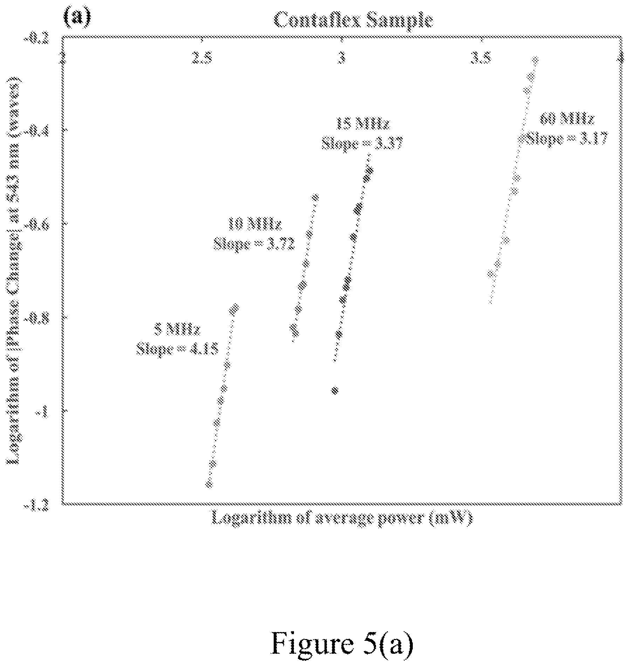

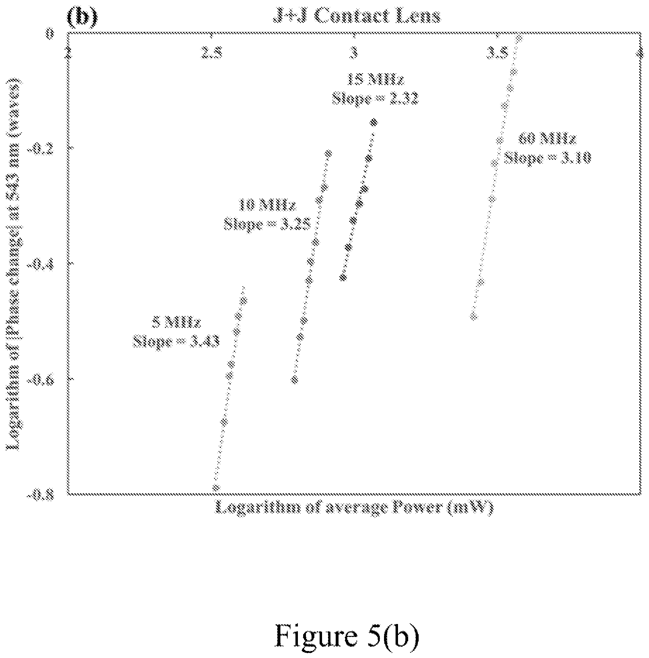

As shown in Fig. 4, the fitting curves derived from the photochemical model and the overlapping effect work well for the low repetition rates at 5 MHz, 10 MHz and 15 MHz but less perfectly for 60 MHz. There are multiple reasons to account for the deviation at 60 MHz. The deviation at high repetition rate may indicate a reduced nonlinearity order from 4 at low number of pulses per spot to around 1 at high number of pulses per spot due to the accumulated linearly absorbing color centers via the incubation reaction. The formation of linearly absorbing defects were also confirmed by an increased absorbance of the laser irradiated area near the UV absorption edge after examining UV-visible transmission spectra. In order to show the decrease in the nonlinearity order we plot the experimental data in a double logarithmic scale. As shown in Figs. 5(a) and 5(b), we plot the logarithm of the induced phase change at four different repetition rate versus the logarithm of the average power in the focal volume. The empirical model was obtained using linear fitting method and thus the order of the multiphoton absorption process was indicated by the linearly fitted trend line gradient. We also observed a decreasing trend line gradient as the repetition rate increases. However, in contrast to the diffraction efficiency used in their paper, which should scale quadratically as the RI change, a direct relationship between the induced phase/RI change and the average power was obtained to determine the order of the multiphoton absorption process. For the Contaflex sample (as shown in Fig. 5(a)), the line gradient decreases from 4.15 at 5 MHz to 3.17 at 60 MHz, which indicates the multiphoton process evolves from a pure four photon absorption process at low number of pulses per spot to a lower order of multiphoton absorption process at high number of pulses per spot due to the formation of larger number of color centers. For the J+J contact lens (as shown in Fig. 5(b)), the trend line gradient follows the decreasing rule as well and slightly reduces from 3.4 at 5 MHz to 3.1 at 60 MHz. The order of multiphoton absorption process keeps decreasing and the overall power dependence is lowered by taking account into both the four photon absorption of the material and the linear absorption of the color centers.

Other than the reduced nonlinearity order at high number of pulses due to color center formation, the deviation at 60 MHz could be attributed to a much higher accumulated temperature rise achieved inside the material after a high-repetition-rate laser exposure. The heat accumulation effect may play a more significant role at a high repetition rate domain and induce the phase change via a distinct photothermal effect which has a power dependency different from that of a photochemical effect. A third explanation for the deviation at 60 MHz could be a saturation effect at high intensities since we noticed the induced phase change cannot increase infinitively and tends to be saturated when the induced phase change reaches a high value. If the saturation factor is not introduced into the theoretical model, the fitting results would be higher than experimental results as the power and repetition rates increase. Therefore, a saturation factor can be incorporated into the photochemical model for the purpose of a better fitting. The saturation factor can be inserted under the material constant $\varepsilon$ if we assume there is a possible limit on the finite number of available molecules, or the capability of the material to redirect the absorbed energy to induce phase change is limited or the depolymerization process is balanced by a recombination effect. On the other hand, the saturation factor can also be attached to the fourth photon absorption coefficient by analogy with the decreasing nonlinear absorption coefficient measured at high laser intensity. We then propose the modified photochemical model can be expressed in the following form by employing a classic saturation factor,

$$\Delta\phi = \frac{\Delta\phi_0}{1+\dfrac{A}{A_{sat}}}$$

$$(5)$$

where $\Delta\phi_0$ is a small signal induced phase change expressed in Eq. (4) and we tried to express $A$ as the induced phase change, the excited molecular density, the average power/intensity, or the peak intensity. The best fitting results for all the repetition rates from 5 MHz to 60 MHz were obtained by denoting $A$ as the average power/intensity and yielded a coefficient of determination $R^2$ greater than 95% for J+J contact lenses and greater than 97% for Contaflex sample at all four repetition rates. However, the modified photochemical model does not work well at 60 MHz when $A$ represents other parameters, including the induced phase change, the excited molecular density, and the peak intensity.

The exact mechanism associating the absorbed pulse energy to RI change is still controversial and could be subj ect to further investigation. The accumulated pulse energy absorbed by the material via multiphoton absorption can excite electrons to a high electronic state to induce direct bond dissociation or the absorbed energy can be transferred from electrons to lattice to heat the polymer matrix and induce subsequent pyrolysis of the polymer. Depolymerization, either a photolysis reaction or a thermal-driven process, usually increases polymer chain volume by random chain scission and direct bond breaking, thus causing monomer formation, internal tensile stress propagation and volume expansion which is confirmed by the shift of the molecular weight to a lower value and the broadening of the polymer molecular weight distribution. After the decomposition of polymer into smaller monomers or oligomers, we assume these smaller fragments may diffuse out from the laser-irradiated area into the surrounding and water molecules can then occupy the empty expanded area due to enhanced hydrolysis, thus resulting in an increasing water content in the laser treated area. Therefore, the negative sign of induced phase/RI change might be attributed to a lower refractive index of water than the sample itself and a reduced polymer density due to hydrodynamic expansion. One potential avenue for further research involves taking advantage of Raman spectroscopy to gain insight into the chemical composition change in the laser-irradiated area and then determine whether the RI change in hydrogel polymers arises from a thermal-induced decomposition process via non-radiative decay or a direct chemical bond breaking procedure via multiphoton ionization or a combined photothermal and photochemical effect.

5. Effect of Repetition Rate on Femtosecond Micromachining Process

The effect of repetition rate has been demonstrated to play an important role in femtosecond micromachining process. The same amount of phase change can be achieved at a lower average power and a faster scan speed by taking advantage of low repetition rate pulses while the damage threshold in terms of single pulse energy maintains almost the same for all the tested repetition rates. Based on both the qualitative and quantitative results, we are able to conclude that the induced phase change depends on the amount of pulse energy absorbed by the material via both the multiphoton absorption and the overlapping effect induced by adjacent pulses. In order to fit the experimental data, we developed a calibration function based on a photochemical model. The induced phase change scales as the power to the fourth order as a consequence of four photon nonlinear absorption at 1035 nm. The optimal repetition rate for femtosecond micromachining contact lenses seems to be around 15 MHz for at least some manufacturing processes, for the reason that the maximum achievable phase change just below the damage is a little bit lower than that obtained at 60 MHz but the average power required is roughly 3 times smaller.

6. Two Photon and Other Models

The above discussion is presented in the context of a four photon regime. For other systems and materials, a two photon regime may be the more appropriate photochemical model, and the following phase change equation may be utilized:

$$\Delta\phi(P,v,S,NA) := \beta \cdot \left( \frac{P^2 \cdot NA}{v \cdot \lambda^3 \cdot S \cdot \tau} \right) \tag{6}$$

where $P$ is the average power, $NA$ is the numerical aperture, and $\tau$ is the pulse duration. Parameter $\beta$ is a constant which contains the second photon absorption coefficient and associates the absorbed energy with the phase change.

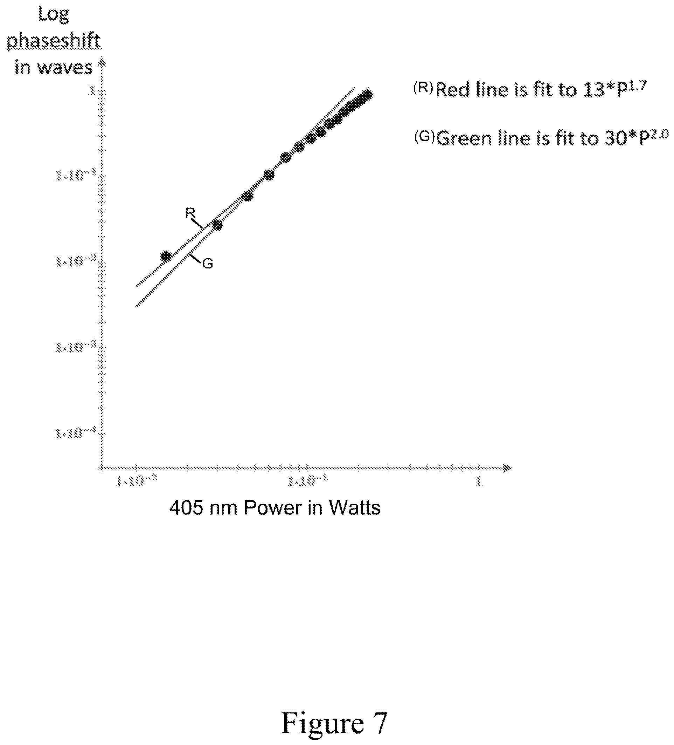

As a general matter, in at least some instances, a two photon model such as the one in eq. (6) may be more appropriate for relatively higher energy writing and a four photon model such as the one in eq. (4) may be more appropraite for relatively lower energy writing. In some instances, the slope of a fitting line in a plot of phase shift versus power on log-log scales may indicate whether a two photon or four photon model is more appropraite. For example the slopes of the lines in Figure 5(a) are approximately 4, indicating use of a four photon model, and the slopes of the lines in Figure 7 are approximately 2, indicating use of a two photon model.

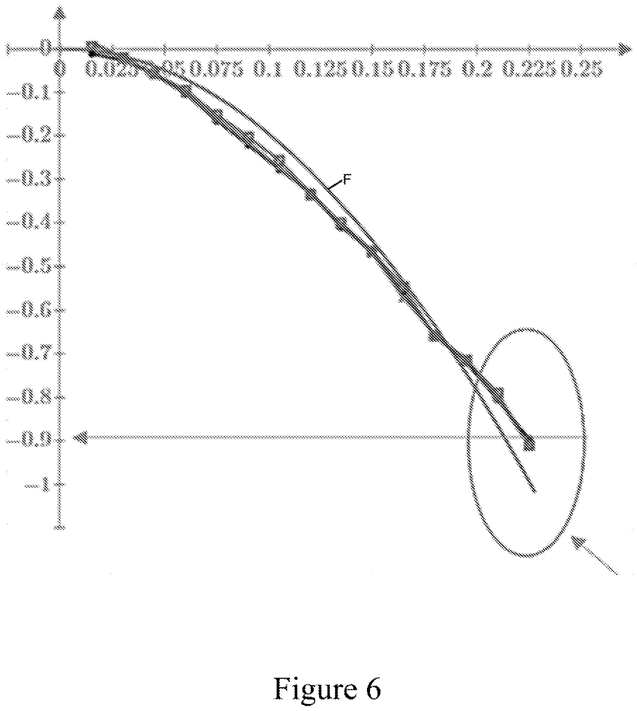

Figure 6 show a plot of phase shifts written into a Contamac 58 sample at 405 nm at different average powers (in Watts), with the line labeled F showing the two photon photochemical model in eq. (6) fitted to the experimental data. In this example, the fitting constant $\beta$ is $5*10^{-24}$ and the maximum phase shift written (measured at 543 nm) below the damage threshold is -0.90.

For some systems and materials, a three or other photon model may be most appropriate, and for a general condition a sum of two-photon, three photon and four photon effects may be most appropriate. For a three photon regime, the following phase change equation may be utilized:

$$\Delta\Phi := \frac{\gamma \cdot P^3 \cdot NA^3}{v^3 \cdot \tau^2 \cdot \lambda^5 \cdot S} \tag{7}$$

where $P$ is the average power, $NA$ is the numerical aperture, and $\tau$ is the pulse duration. Parameter $\gamma$ is a constant which contains the third photon absorption coefficient and associates the absorbed energy with the phase change.

For an Nth photon regime, the following phase change equation may be utilized:

$$\Delta\Phi := \frac{\gamma \cdot P^N \cdot NA^{2 \cdot N - 3}}{v^{N-1} \cdot \tau^{N-1} \cdot \lambda^{2 \cdot N - 1} \cdot S} \tag{8}$$

where $P$ is the average power, $NA$ is the numerical aperture, and $\tau$ is the pulse duration. Parameter $\gamma$ is a constant which contains the Nth photon absorption coefficient and associates the absorbed energy with the phase change.

For a general condition including a sum of two-photon, three-photon, and four-photon effects, the following phase change equation may be utilized:

$$\Delta\Phi := \beta \cdot \frac{P^2 \cdot NA}{v \cdot \tau \cdot \lambda^3 \cdot S} + \gamma \cdot \frac{P^3 \cdot NA^3}{v^2 \cdot \tau^2 \cdot \lambda^5 \cdot S} + \delta \cdot \frac{P^4 \cdot NA^5}{v^3 \cdot \tau^3 \cdot \lambda^7 \cdot S} \tag{9}$$

where P is the average power, $NA$ is the numerical aperture, and $\tau$ is the pulse duration. In this instance, parameters $\beta$, $\gamma$, and $\delta$ are constants which contain the second, third, and fouth photon absorption coefficients respectively.

### Scalable Manufacturing with LIRIC

Using the information derived from the photochemical models and experiments discussed above, including information about the maximum phase shift obtainable in a given material at applicable scanning speeds, numerical apertures, and repetition rates, commercial scale LIRIC systems and methods with optimal throughput and other efficiencies can be designed and implemented.

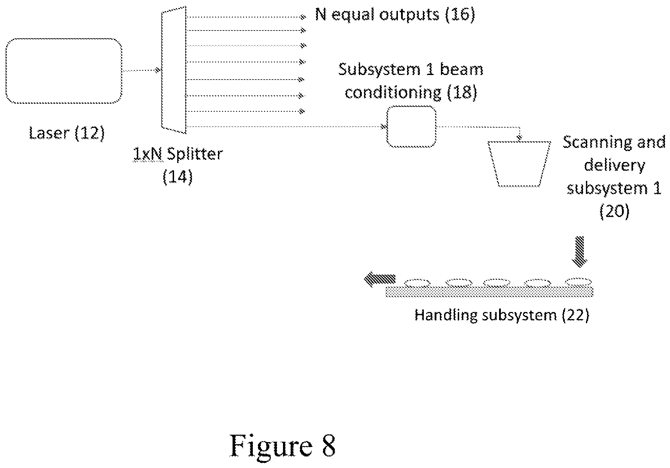

Figure 8 shows one example of a LIRIC multi-head writing system that may be used for ophthalmic materials such as contract lenses or intra-ocular lenses. The system of Figure 8 includes a laser 12 and a splitter 14, splitting the laser's beam into N equal outputs 16. Each output may be associated with conditioning, scanning, and delivery subsystems, although, in Figure 8, only one beam conditioning subsystem 18 and one scanning & delivery subsystem 20 are specifically shown. As also schematically shown in Figure 8, a handling subsystem 22 may be included to facilitate loading, positioning & aligning the ophthalmic devices relative to the writing heads, and unloading in an efficient manner to further help with overall throughput of the laser writing system.

Various lasers may be used for the writing system. Non-limiting examples include short pulsed lasers (such as femtosecond lasers with a pulsewidth less than 350 fs and a repetition rate (tunable or fixed repetition rate) in the range of 1 to 60 MHz), operating in the range of 340 nm to 1100 nm (e.g. near 405 nm, 517 nm, 800 nm, or 1035 nm).

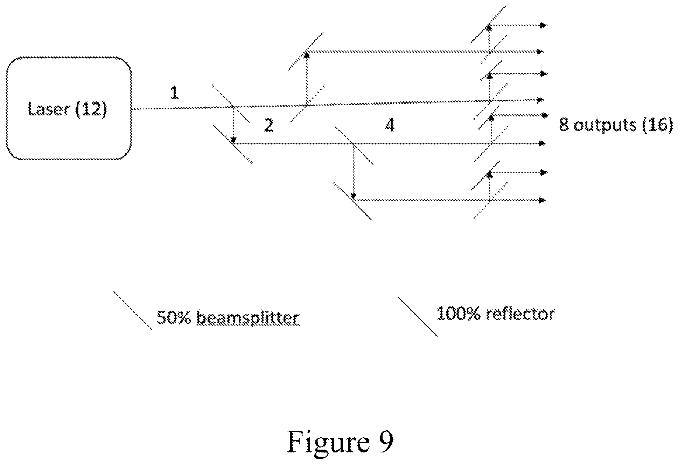

Figure 9 shows one example of a beam power splitter that that may be used in the LIRIC multi-head writing system of Figure 1. In Figure 9, 50% beam-splitters (illustrated by dashed diagonal lines) and 100% reflectors (illustrated by solid diagonal lines) split the relatively high-power beam of laser 12 into eight equal lower power beams 16. In other examples, the splitter may split the laser beam into unequal lower power beams.

Returning to Figure 8, at the beam conditioning subsystem 18, the lower power laser beam may undergo, for example, dispersion compensation, beam sizing, power control (e.g. using acousto-optic or electro-optic components), ellipticity control, and/or power monitoring.

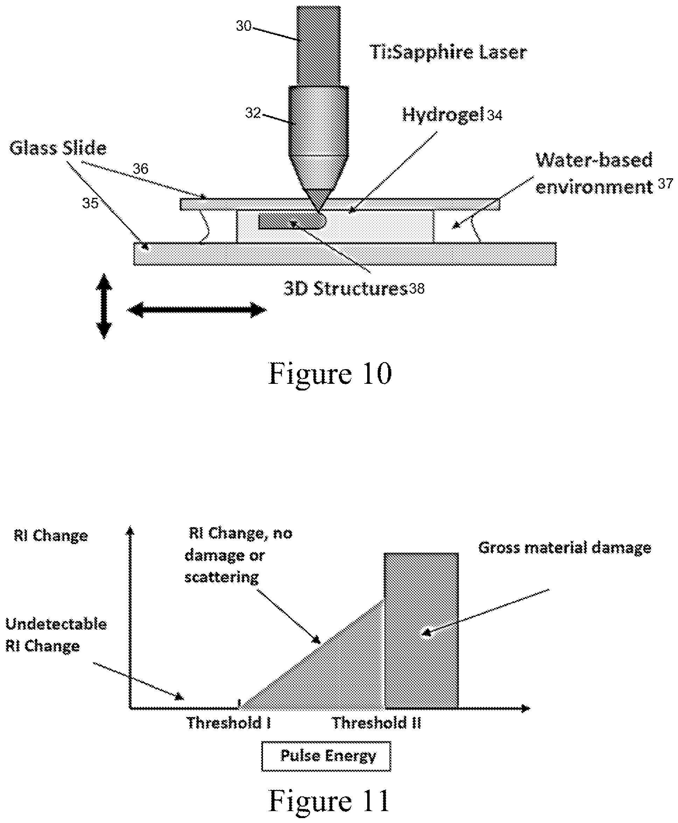

Figure 10 shows one example of a scanning and delivery subsystem that may be used in the LIRIC multi-head writing system of Figure 8. In Figure 10, a laser beam 30 (in this example, from a Ti:Sapphire laser) is sent through an objective 32 to form a diffraction-limited spot inside an ophthalmic material (in this example, a hydrogel 34 held between two glass slides 35 and 36 in a water based environment 37), thereby forming 3D structures 38. Although not shown, the sample may be mounted on a translation stage for axial scanning along X and Y axes, and the objective may be translated along a Z-axis for vertical translation, allowing precise writing in the material in three dimensions, Any suitable scanning system may be utilized, including, without limitation, high speed XYZ translation stages, high speed galvanometer scanning systems, and shaker scanners (such as described in U.S. Patent Application Publication No. 2016/0144580 published May 26, 2016 to Wayne H,. Knox et al.). In some instances, the scanning speed may be in the range of 1 mm/sec to 10 meters/sec.

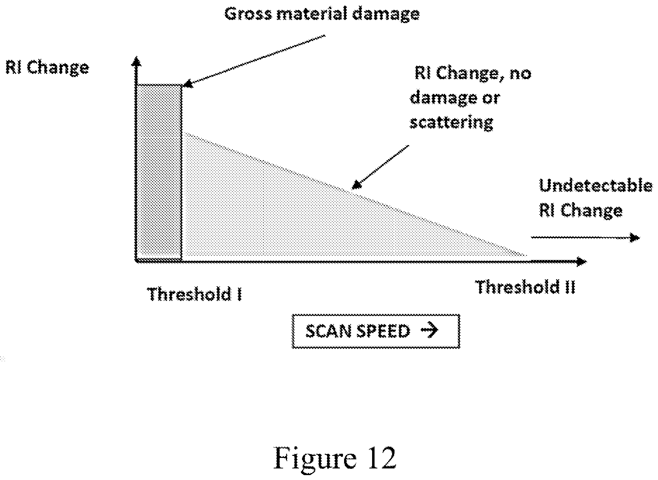

The scanning and delivery subsystem may deliver short laser pulses of sufficient energy (e.g. above a minimal threshold but below a damage threshold, as shown in Figure 11) and at sufficient scan speeds (e.g. above a damage threshold but below an upper speed threshold, as shown in Figure 12) to cause a nonlinear absorption of photons (typically multi-photon absorption), leading to a change in the refractive index of the material at the focus point. In Figures 11 and 12, the damage threshold may reflect a threshold in which a degradation in optical quality of the device is detectable. Moreover, the region of the material just outside the focal region is minimally affected by the laser light. Accordingly, select regions of an optical, polymeric material can be modified with a laser resulting in a change in the refractive index in these regions. The irradiated regions may exhibit no significant differences in the Raman spectrum with respect to the non-irradiated regions. Also, the irradiated regions may exhibit little or no scattering loss, which means that the structures formed in the irradiated regions are not clearly visible under appropriate magnification without contrast enhancement.

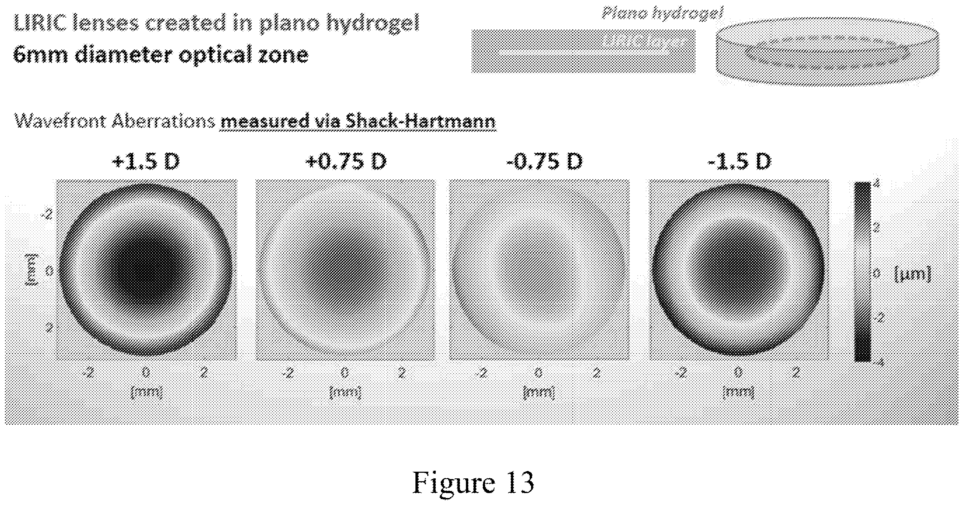

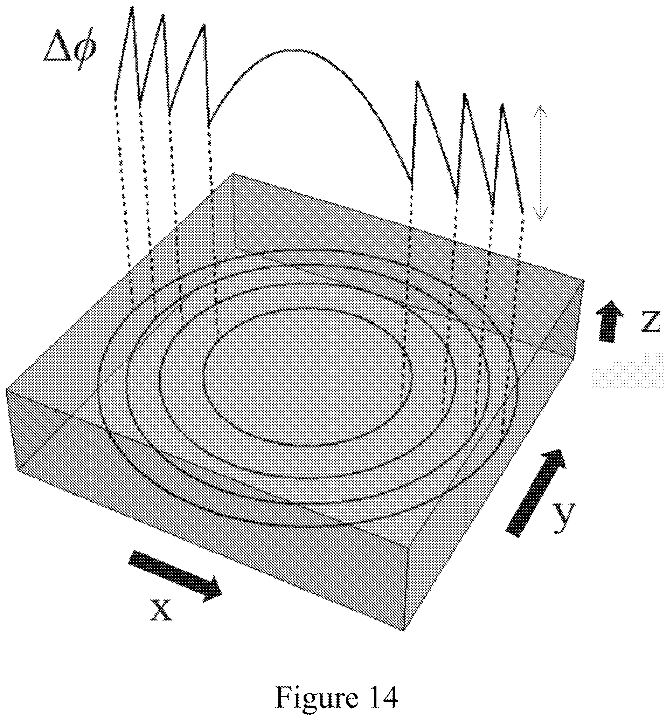

The writing system may be utilized to create lenses or other optical constructs in the interior of the material. For example, Figure 13 shows LIRIC lenses written into the interior of hydrogels, and Figure 14 shows a Fresnel-type phase wrapped optical lens written into the interior of a plano hydrogel. Similar lenses or other optical constructs may also be written into contact lenses to change their optical properties. Depending on the maximum phase shift required, the lens or other optical construct can be written into the material in a single layer or in multiple layers. U.S. Patent 8,932,352, issued January 13, 2015 to Wayne H. Knox et al. for an "Optical Material and Method for Modifying the Refractive Index" and U.S. Patent 9,144,491, issued September 29, 2015 to Wayne H. Knox et al. for a "Method for Modifying the Refractive Index of an Optical Material," describe additional examples of gratings and other optical constructs that may be written into materials.

Using the information derived from the photochemical models discussed above, manufacturing systems like the one of Figures 8-10 can be designed and configured for optimal scale and throughput. There are numerous inputs that potentially impact on the throughput of a LIRIC system, and that could be accounted for as inputs into an optimization model, including, without limitation, laser specifications, scanner specifications, material characteristics, and desired design for the LIRIC devices. Of course, some of these inputs, such as certain desired design characteristics for the device, may not be subject to variation for purposes of manufacturing optimization, while other variables, including certain laser specifications, are more readily subject to variation in order to optimize the LIRIC manufacturing process.

1. Laser Specifications

Laser specification inputs may include, without limitation, average laser power, pulsewidth, wavelength, repetition rate, lens NA numerical aperture, system power throughput, and number of device writing heads. Example pulsewidths include femtosecond scale pulsewidths, and, in some examples, pulsewidths less than 350 fs. Example repetition rates include repetition rates in the range of 1-60 MHz. Example wavelengths include wavelengths in the range of 340 nm to 1100 nm, including wavelengths near 405 nm, 517 nm, 800 nm, and 1035 nm. Example lens NA include NA's between 0.19 and 1.0.

2. Scanner Specifications

Scanner specifications inputs may include, without limitation, maximum scanning speed, slow axis resolution (line spacing), turnaround (blanking) time, full field size, and scan pattern (e.g. raster, box, spiral, etc.). Example scanning speeds include speeds in the range from 1 mm/sec to 10 meters/sec.

3. Material Characteristics

Material characteristic inputs may include, without limitation, non-linear absorption coefficient of the material (such as one or more of the $2^{nd}$ order limit, $4^{th}$ order limit, and mixed $2^{nd}$ through $4^{th}$ order terms), as well as the maximum phase shift obtainable (determined at applicable scanning speeds, numerical apertures, repetition rates, or other factors).

In one example, a first step to determining material characteristics is to measure written phase shifts in the material of interest as a function of, for example, average power and scan speed. Phase vs. power can be plotted on log-log scales to determine slope of nonlinear processes in small signal regime (below saturation). The determined slope may be indicative of the applicable photochemical model for the material of interest (e.g. a slope close to 2 may be indicative of a two photon model, a slope close to 4 may be indicative of a four photon model, etc.). The applicable photochemical models (e.g. the two and/or four photon regime models identified above) may be fit to the data, and a maximum phase shift just below the damage threshold may be identified. This information may be subsequently used to establish a range of writing powers at desired scan speeds. It is noted that, while the best results for at least some systems and methods are obtained when the one-photon absorption is minimized, a small amount of one-photon absorption is tolerable even in those instances.

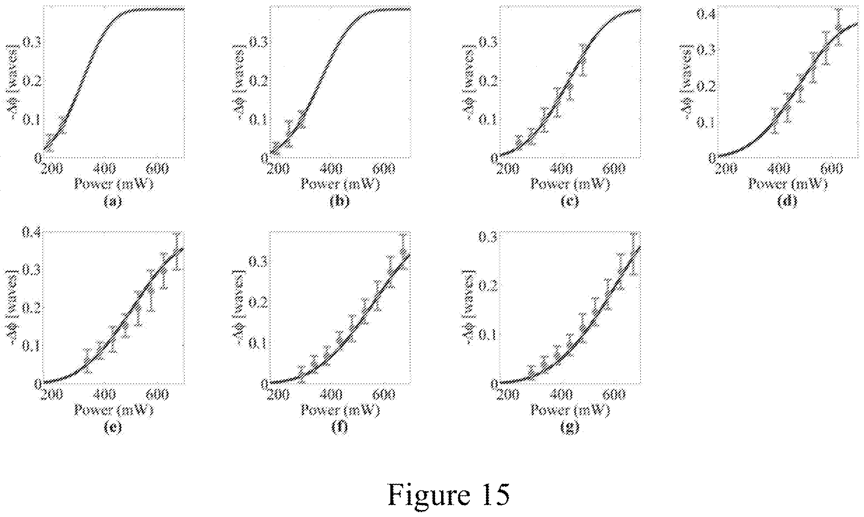

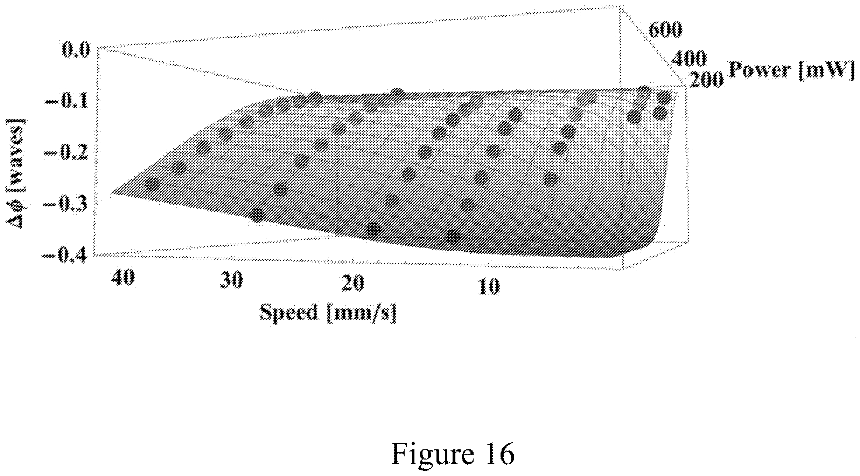

A specific example in the two photon regime is provided at Figures 6-7. A specific example in the four photon regime of measuring written phase shifts in the material of interest as a function of both average power and scan speed is provided at Figures 15-16, which could be analyzed in a similar manner as the data in Figures 6-7. Figure 15 illustrates data plotting phase change as a function of power and speed obtained from LIRIC experiments performed in Johnson & Johnson Acuvue2 contacts in response to 100 fs laser pulses at a repetition rate of 80 MHz and 800 nm, for speeds of (a) 3 mm/s, (b) 5 mm/s, (c) 10 mm/s, (d) 15 mm/s, (e) 20 mm/s, (f) 30 mm/s, and (g) 40 mm/s, while Figure 16 is a three-dimensional visualization of the data in Figure 15. Another example in the four photon regime, but measuring phase shifts as a function of average power and repetition rate is provided at Figures 4-5.

### 4. Device Design Details

Device design inputs may include, without limitation, diameter of the optical device to be written, the line spacing required (e.g. to ensure smooth phase shifts that produce no diffraction spots), and details impacting on the maximum phase shift required for the device (e.g. the phase wrapping profile, intended optical power, etc.).

### 5. Scan Layers Required

The number of scan layers required for the LIRIC process will impact on the time required to write the device. The number of scan layers required is a function of both the maximum phase shift required by the device's specification and the maximum phase shift produced in the device material at the relevant laser and scanner parameters. The number of layers required is the max phase shift required for the device divided by the max phase shift produced in the device material at the relevant laser and scanner parameters.

### 6. Estimated Writing Time

Estimated writing time for a device is a function of how many layers need to be written, as well as several other variables, including device width or diameter, line spacing, number of lines (device width divided by line spacing), scan speed, and scanner efficiency (to account for dead time). Time to write a device can be estimated by,

$$T := \frac{W^2 \cdot N_{layers}}{S \cdot \Delta \cdot \eta}$$

(10)

in which $W$ is device width in mm, $N_{lines}$ is the number of lines, $S$ is the scan speed in mm/s, and $\eta$ is scanner efficiency.

### 7. Number of Writing Heads

The number of writing heads that can be incorporated into the multi-head writing system is a function of the laser's maximum power, the maximum power required to write a one layer phase shift in the device, and a system loss factor (typically about 50%). Number of writing heads that could be supported by the system can be estimated by,

$$N_{heads} = (P_{max} * F) / P_{max\text{-}phaseshift}$$

(11)

in which $P_{max}$ is the laser's maximum power, $P_{max\text{-}phaseshift}$ is the maximum power required to write a one layer phase shift in the device, and F is the system loss factor. As one example, for a 517 nm laser with a maximum power of 10W, a 50% system loss factor, and a 0.5W per device requirement to achieve a single layer max phase-shift, it is estimated that the system could support 10 heads with one single laser source.

### 8. LIRIC Optimization For Multi-Head Writing Systems

We have discovered that laser reptition rate can have a surpsingly significant impact on several aspects of the LIRIC writing process. Surprisingly, relatively low reptition rates can be used to achieve significant phase changes at relatively low power requirements. This can be visualized, for example, using Figure 4(a), in which the 5 - 15 MHz processes achieved significant phase change values at much lower powers than the corresponding 60 MHz process. One potential trade off to this is that, in at least some instances, lower reptition rate processes can reach a damage threshold of the subject material at lower phase change and power values, potentially requiring that the device be written in more layers than would necessarily be required with higher repetition rate processes. This can also be visualized using Figure 4(a), in which it can be seen that the 5 - 15 MHz processes reach a maximum phase change before material damage (the upper end of the fitting lines for those processes) before the corresponding 60 MHz process does.

Taking into account these factors, as well as other factors in the LIRIC writing process discussed above, can allow for optimization of manufacturing throughput. One example is presented below in table 1, which assumes a LIRIC process utilizing a 30 W, 1035 nm laser having the same average power at all reptition rates, with the system having a 50% system loss and being used to manufacture a device requiring a max phase shift of 1 wave.

Table 1

|  | 60 MHz | 5 MHz |
|---|---|---|
| $P_{max}$ (W) | 3.75 | 0.45 |
| $\Delta\Phi_{max}$ | 1.0 | 0.35 |
| $N_{heads}$ | 4 | 33 |
| $N_{layers}$ | 1 | 3 |

As can be seen from table 1, the 5 MHz process could power over 30 writing heads, 8.25 times as many as the 60 MHz process can power. However, the trade off is that the 5 MHz process would require three times the number of layers to be written as the 60 MHz process, roughly tripling the writing time rquired for each device. In this simple example, although it would require three times the number of layers to be written, the much larger number of wrting heads supportable by the 5 MHz process would result in that system having a throughput of 2.75x greater than the 60 MHz process.

**Claims**

1. A method of designing a laser writing system for modifying a plurality of ophthalmic devices, the method comprising:

   (a) determining at least one material characteristic of the ophthalmic devices, determined over a range of laser writing system parameters;
   (b) determining at least one design characteristic of the ophthalmic device; and
   (c) using at least the determined material and design characteristics, configuring at least one system parameter of the laser writing system to optimize throughput of the laser writing system, the laser writing system comprising:

      (i) a laser configured to generate a laser beam,
      (ii) a splitter configured to split the laser beam into a plurality of outputs, and
      (iii) a plurality of writing heads, each writing head configured to direct at least one of the outputs to an ophthalmic device to write one or more localized refractive index modifications into the ophthalmic device;

   wherein the at least one design characteristic includes a maximum phase shift designed for the ophthalmic device to be written in the ophthalmic device with the laser writing system without causing optical damage in the ophthalmic devices;
   wherein the at least one material characteristic includes at least a first damage threshold for an induced single layer optical phase shift of the ophthalmic device at a first laser repetition rate, and a second damage threshold for an induced single layer optical phase shift of the ophthalmic device at a second laser repetition rate; and
   wherein optimizing throughput of the laser writing system comprises determining an optimal number of writing layers for modifying the ophthalmic devices in combination with an optimal number of writing heads of the laser writing system for optimizing throughput of the laser writing system and obtaining the maximum phase shift designed for the ophthalmic device without causing optical damage in the ophthalmic devices.

2. The method of claim 1, wherein the range of laser writing system parameters used for determining the at least one material characteristic includes at least one of a power range, a scan speed range, a laser repetition rate range, and a range of focusing lens numerical aperture.

3. The method of claim 2, wherein the range of laser writing system parameters used for determining the at least one material characteristic includes at least two of the power range, the scan speed range, the laser repetition rate range, and the focusing lens numerical aperture.

4. The method of claim 2 or 3, wherein the determined material characteristics also include at least one fitting parameter of a quantitative model relating an induced phase shift in the ophthalmic device to a configuration of the laser writing system.

5. The method of claim 4, wherein the determined material characteristics also include a multiphoton order of the quantitative model, wherein preferably the quantitative model further comprises a saturation factor.

6. The method of claim 5, wherein the quantitative model is a two photon regime model comprising:

$$\Delta\phi\left(P,v,S,NA\right):=\beta\cdot\left(\frac{P^2 \cdot NA}{v\cdot\lambda^3\cdot S\cdot\tau}\right)$$

wherein $\Delta\phi$ is a single layer written phase shift, $P$ is an average power, $NA$ is a numerical aperture, v is a laser repitition rate, $\lambda$ is a laser wavelength, $S$ is a scan speed, $\tau$ is a pulse duration, and $\beta$ is the fitting parameter; or wherein the quantitative model is a three photon regime model comprising:

$$\Delta\Phi:=\frac{\gamma\cdot P^3 \cdot NA^3}{v^2\cdot\tau^2\cdot\lambda^5\cdot S}$$

wherein $\Delta\phi$ is a single layer written phase shift, $P$ is an average power, $NA$ is a numerical aperture, v is a laser repitition rate, $\lambda$ is a laser wavelength, $S$ is a scan speed, $\tau$ is a pulse duration, and $\gamma$ is the fitting parameter; or wherein the quantitative model is a four photon regime model comprising:

$$\Delta\phi = \gamma \cdot\frac{P^4 \cdot NA^5}{v^3\cdot\tau^3\cdot S\cdot\lambda^7}$$

wherein $\Delta\phi$ is a written single layer phase shift, $P$ is an average power, $NA$ is a numerical aperture, v is a laser repitition rate, $\lambda$ is a laser wavelength, $S$ is a scan speed, $\tau$ is a pulse duration, and $\gamma$ is the fitting parameter.

7. The method of claim 5, wherein the quantitative model is an $N^{th}$ photon regime model comprising:

$$\Delta\Phi:=\frac{\gamma\cdot P^N \cdot NA^{2\cdot N-3}}{v^{N-1}\cdot\tau^{N-1}\cdot\lambda^{2\cdot N-1}\cdot S}$$

wherein $\Delta\phi$ is a single layer written phase shift, $P$ is an average power, $NA$ is a numerical aperture, v is a laser repitition rate, $\lambda$ is a laser wavelength, $S$ is a scan speed, $\tau$ is a pulse duration, and $\gamma$ is the fitting parameter.

8. The method of claim 4, wherein the quantitative model comprises a sum of a plurality of multi-photon regime models,

preferably wherein the quantitative model comprises:

$$\Delta\Phi:=\beta\cdot\frac{P^2 \cdot NA}{v\cdot\tau\cdot\lambda^3\cdot S}+\gamma\cdot\frac{P^3 \cdot NA^3}{v^2\cdot\tau^2\cdot\lambda^5\cdot S}+\delta\cdot\frac{P^4 \cdot NA^5}{v^3\cdot\tau^3\cdot\lambda^7\cdot S}$$

wherein $\Delta\phi$ is a single layer written phase shift, $P$ is an average power, $NA$ is a numerical aperture, v is a laser

repitition rate, $\lambda$ is a laser wavelength, $S$ is a scan speed, $\tau$ is a pulse duration, $\beta$ is a second order fitting parameter, $\gamma$ is a third order fitting parameter, and $\delta$ is a fourth order fitting parameter.

9. The method of any one of claims 1 to 8, wherein optimizing throughput of the laser writing system comprises determining at least one of an optimal laser repetition rate, laser wavelength, laser pulsewidth, and scan speed for optimizing throughput of the laser writing system; and
preferably further comprising determining a line spacing characteristic of the ophthalmic device.

10. A method of laser writing for writing localized refractive index changes into ophthalmic devices with optimized throughput while obtaining a maximum phase shift designed for the ophthalmic devices without causing optical damage in the ophthalmic devices, the method designing a laser writing system by the method of any of claims 1-9, the method of laser writing further comprising:

   (a) generating a pulsed laser beam in the laser writing system;
   (b) splitting the pulsed laser beam into a plurality of outputs, at least some of the outputs each associated with one of an optimal number of writing heads in the laser writing system; and
   (c) at each of the optimal number of writing heads, scanning the pulsed laser beam relative to an ophthalmic device to write an optimal number of writing layers having one or more refractive index changes into the ophthalmic device;

   wherein the optimal number of writing heads and optimal number of writing layers are determined in combination for optimizing throughput of the laser writing system while obtaining the maximum phase shift designed for the ophthalmic devices without causing optical damage in the ophthalmic devices.

11. The method of claim 10, wherein step (a) comprises generating a pulsed laser beam having a pulsewidth less than 350 femtoseconds and a repetition rate between 1 and 60 MHz;

   wherein preferably the generated pulsed laser beam has a wavelength in the range of 340 nm to 1100 nm;
   wherein preferably the generated pulsed laser beam has a wavelength in the range of 515 nm to 520 nm, or 1030 nm to 1040 nm, or 400 nm to 410 nm, or 795 nm to 805 nm; and
   wherein preferably in step (b) the laser beam is split into 2 to 64 outputs.

12. The method of claim 10 or 11, wherein the ophthalmic devices are contact lenses or intraocular lenses, or hydrogel corneal implants.

13. The method of any one of claims 10 to 12,

   wherein step (a) comprises generating a pulsed laser beam having a wavelength in the range of 1030 nm to 1040 nm, a pulsewidth less than 350 femtoseconds, and a repetition rate less than 20 MHz; and
   wherein the laser writing system is operated to induce a single writing layer phase shift of at least 0.3 waves at an average power at the writing head of less than 1500 mW and a scan speed greater than 100 mm/s.

14. The method of claim 13, wherein the ophthalmic device is a hydrogel material having a four photon absorption parameter in the range of 1e-59 $m^6 \cdot$waves/($W^4 \cdot$s) to 3e-59 $m^6 \cdot$waves/($W^4 \cdot$s) in the equation:

$$\Delta\phi = \gamma \cdot \frac{P^4 \cdot NA^5}{\upsilon^3 \cdot \tau^3 \cdot S \cdot \lambda^7}$$

wherein $\Delta\phi$ is a written single layer phase shift, $P$ is an average power of the pulsed laser beam at the writing head, $NA$ is a numerical aperture of the writing head, $\upsilon$ is a laser repitition rate, $\lambda$ is a laser wavelength, $S$ is a scan speed, $\tau$ is a laser pulse duration, and $\gamma$ is the four photon absorption parameter.

**Patentansprüche**

1. Verfahren zum Entwerfen eines Laserschreibsystems zum Modifizieren einer Vielzahl von ophthalmischen Vorrichtungen, wobei das Verfahren umfasst:

(a) Bestimmung mindestens einer Materialeigenschaft der ophthalmischen Geräte, die über einen Bereich von Parametern des Laserschreibsystems bestimmt wird;

(b) Bestimmung mindestens eines Konstruktionsmerkmals der ophthalmischen Vorrichtung; und

(c) Konfigurieren mindestens eines Systemparameters des Laserschreibsystems unter Verwendung mindestens der ermittelten Material- und Konstruktionseigenschaften, um den Durchsatz des Laserschreibsystems zu optimieren, wobei das Laserschreibsystem umfasst:

(i) einen Laser, der so konfiguriert ist, dass er einen Laserstrahl erzeugt,

(ii) einen Splitter, der so konfiguriert ist, dass er den Laserstrahl in eine Vielzahl von Ausgängen aufteilt, und

(iii) eine Vielzahl von Schreibköpfen, wobei jeder Schreibkopf so konfiguriert ist, dass er mindestens einen der Ausgänge auf eine ophthalmische Vorrichtung richtet, um eine oder mehrere lokalisierte Brechungsindexänderungen in die ophthalmische Vorrichtung zu schreiben;

wobei das mindestens eine Konstruktionsmerkmal eine maximale Phasenverschiebung umfasst, die für die ophthalmische Vorrichtung so ausgelegt ist, dass sie mit dem Laserschreibsystem in die ophthalmische Vorrichtung geschrieben werden kann, ohne optische Schäden in den ophthalmischen Vorrichtungen zu verursachen;

wobei die mindestens eine Materialeigenschaft mindestens eine erste Schadensschwelle für eine induzierte optische Einzelschicht-Phasenverschiebung der ophthalmischen Vorrichtung bei einer ersten Laserwiederholrate und eine zweite Schadensschwelle für eine induzierte optische Einzelschicht-Phasenverschiebung der ophthalmischen Vorrichtung bei einer zweiten Laserwiederholrate umfasst; und

wobei das Optimieren des Durchsatzes des Laserschreibsystems das Bestimmen einer optimalen Anzahl von Schreibschichten zum Modifizieren der ophthalmischen Vorrichtungen in Kombination mit einer optimalen Anzahl von Schreibköpfen des Laserschreibsystems umfasst, um den Durchsatz des Laserschreibsystems zu optimieren und die maximale Phasenverschiebung zu erhalten, die für die ophthalmische Vorrichtung ausgelegt ist, ohne optische Schäden in den ophthalmischen Vorrichtungen zu verursachen.

2. Verfahren nach Anspruch 1, wobei der Bereich der Parameter des Laserschreibsystems, die zur Bestimmung der mindestens einen Materialeigenschaft verwendet werden, mindestens einen Leistungsbereich, einen Bereich der Abtastgeschwindigkeit, einen Bereich der Laserwiederholrate und einen Bereich der numerischen Apertur der Fokussierlinse umfasst.

3. Verfahren nach Anspruch 2, wobei der Bereich der Parameter des Laserschreibsystems, die zur Bestimmung der mindestens einen Materialeigenschaft verwendet werden, mindestens zwei aus dem Leistungsbereich, dem Bereich der Abtastgeschwindigkeit, dem Bereich der Laserwiederholrate und der numerischen Apertur der Fokussierlinse umfasst.

4. Verfahren nach Anspruch 2 oder 3, wobei die ermittelten Materialeigenschaften auch mindestens einen Anpassungsparameter eines quantitativen Modells umfassen, das eine induzierte Phasenverschiebung in der ophthalmischen Vorrichtung mit einer Konfiguration des Laserschreibsystems in Beziehung setzt.

5. Verfahren nach Anspruch 4, wobei die ermittelten Materialeigenschaften auch eine Mehrphotonenordnung des quantitativen Modells umfassen, wobei das quantitative Modell vorzugsweise zusätzlich einen Sättigungsfaktor umfasst.

6. Verfahren nach Anspruch 5, wobei das quantitative Modell ein Zwei-Photonen-Modell ist, das Folgendes umfasst:

$$\Delta\phi\left(P,v,S,NA\right) := \beta \cdot \left(\frac{P^2 \cdot NA}{v \cdot \lambda^3 \cdot S \cdot \tau}\right)$$

wobei $\Delta\phi$ eine einschichtig geschriebene Phasenverschiebung ist, $P$ eine mittlere Leistung ist, $NA$ eine numerische Apertur ist, v eine Laserwiederholrate ist, $\lambda$ eine Laserwellenlänge ist, $S$ eine Abtastgeschwindigkeit ist, $\tau$ eine Pulsdauer ist und $\beta$ der Anpassungsparameter ist; oder

wobei das quantitative Modell ein Drei-Photonen-Regime-Modell ist, das Folgendes umfasst

$$\Delta\Phi := \frac{\gamma \cdot P^3 \cdot NA^3}{v^2 \cdot \tau^2 \cdot \lambda^5 \cdot S}$$

wobei $\Delta\phi$ eine einschichtig geschriebene Phasenverschiebung ist, $P$ eine mittlere Leistung ist, $NA$ eine numerische Apertur ist, v eine Laserwiederholrate ist, $\lambda$ eine Laserwellenlänge ist, $S$ eine Abtastgeschwindigkeit ist, $\tau$ eine Pulsdauer ist und $\gamma$ der Anpassungsparameter ist; oder

wobei es sich bei dem quantitativen Modell um ein Vier-Photonen-Regime-Modell handelt, das Folgendes umfasst:

$$\Delta\phi = \gamma \cdot \frac{P^4 \cdot NA^5}{v^3 \cdot \tau^3 \cdot S \cdot \lambda^7}$$

wobei $\Delta\phi$ eine einschichtig geschriebene Phasenverschiebung ist, $P$ eine mittlere Leistung ist, $NA$ eine numerische Apertur ist, v eine Laser-Wiederholrate ist, $\lambda$ eine Laser-Wellenlänge ist, $S$ eine Abtastgeschwindigkeit ist, $t$ eine Pulsdauer ist und $\gamma$ der Anpassungsparameter ist.

7. Verfahren nach Anspruch 5, wobei das quantitative Modell ein $N^{th}$-Photonen-Regime-Modell ist, das Folgendes umfasst:

$$\Delta\Phi := \frac{\gamma \cdot P^N \cdot NA^{2 \cdot N - 3}}{v^{N-1} \cdot \tau^{N-1} \cdot \lambda^{2 \cdot N - 1} \cdot S}$$

wobei $\Delta\phi$ eine einschichtig geschriebene Phasenverschiebung ist, $P$ eine mittlere Leistung ist, $NA$ eine numerische Apertur ist, v eine Laserwiederholrate ist, $\lambda$ eine Laserwellenlänge ist, $S$ eine Abtastgeschwindigkeit ist, $t$ eine Pulsdauer ist und $\gamma$ der Anpassungsparameter ist.

8. Verfahren nach Anspruch 4, wobei das quantitative Modell eine Summe einer Vielzahl von Multi-Photonen-Regime-Modellen umfasst,

wobei das quantitative Modell vorzugsweise Folgendes umfasst:

$$\Delta\Phi := \beta \cdot \frac{P^2 \cdot NA}{v \cdot \tau \cdot \lambda^3 \cdot S} + \gamma \cdot \frac{P^3 \cdot NA^3}{v^2 \cdot \tau^2 \cdot \lambda^5 \cdot S} + \delta \cdot \frac{P^4 \cdot NA^5}{v^3 \cdot \tau^3 \cdot \lambda^7 \cdot S}$$

wobei $\Delta\phi$ eine einschichtig geschriebene Phasenverschiebung ist, $P$ eine mittlere Leistung ist, $NA$ eine numerische Apertur ist, v eine Laserwiederholrate ist, $\lambda$ eine Laserwellenlänge ist, $S$ eine Abtastgeschwindigkeit ist, $t$ eine Pulsdauer ist, $\beta$ ein Anpassungsparameter zweiter Ordnung ist, $\gamma$ ein Anpassungsparameter dritter Ordnung ist und $\delta$ ein Anpassungsparameter vierter Ordnung ist.

9. Verfahren nach einem der Ansprüche 1 bis 8, wobei das Optimieren des Durchsatzes des Laserschreibsystems das Bestimmen mindestens einer optimalen Laserwiederholrate, Laserwellenlänge, Laserpulsbreite und Abtastgeschwindigkeit zur Optimierung des Durchsatzes des Laserschreibsystems umfasst; und

vorzugsweise umfasst dies auch die Bestimmung einer Linienabstandscharakteristik des ophthalmischen Geräts.

10. Laserschreibverfahren zum Schreiben von lokalisierten Brechungsindexänderungen in ophthalmische Vorrichtungen mit optimiertem Durchsatz bei gleichzeitiger Erzielung einer maximalen Phasenverschiebung, die für die ophthalmischen Vorrichtungen ausgelegt ist, ohne optische Schäden in den ophthalmischen Vorrichtungen zu verursachen, wobei das Verfahren ein Laserschreibsystem nach einem der Ansprüche 1 bis 9 entwirft, wobei das Laserschreibverfahren ferner umfasst:

(a) Erzeugung eines gepulsten Laserstrahls im Laserschreibsystem;
(b) Aufspalten des gepulsten Laserstrahls in eine Vielzahl von Ausgängen, wobei mindestens einige der Ausgänge jeweils einem einer optimalen Anzahl von Schreibköpfen in dem Laserschreibsystem zugeordnet sind;

und

(c) Scannen des gepulsten Laserstrahls relativ zu einer ophthalmischen Vorrichtung an jedem der optimalen Anzahl von Schreibköpfen, um eine optimale Anzahl von Schreibschichten mit einer oder mehreren Brechungsindexänderungen in die ophthalmische Vorrichtung zu schreiben;

wobei die optimale Anzahl von Schreibköpfen und die optimale Anzahl von Schreibschichten in Kombination bestimmt werden, um den Durchsatz des Laserschreibsystems zu optimieren und gleichzeitig die maximale Phasenverschiebung zu erhalten, die für die ophthalmischen Geräte ausgelegt ist, ohne optische Schäden in den ophthalmischen Geräten zu verursachen.

**11.** Verfahren nach Anspruch 10, wobei Schritt (a) das Erzeugen eines gepulsten Laserstrahls mit einer Impulsbreite von weniger als 350 Femtosekunden und einer Wiederholrate zwischen 1 und 60 MHz umfasst;

wobei der erzeugte gepulste Laserstrahl vorzugsweise eine Wellenlänge im Bereich von 340 nm bis 1100 nm aufweist;
wobei der erzeugte gepulste Laserstrahl vorzugsweise eine Wellenlänge im Bereich von 515 nm bis 520 nm, oder 1030 nm bis 1040 nm, oder 400 nm bis 410 nm, oder 795 nm bis 805 nm aufweist; und
wobei vorzugsweise in Schritt (b) der Laserstrahl in 2 bis 64 Ausgänge aufgeteilt wird.

**12.** Verfahren nach Anspruch 10 oder 11, wobei die ophthalmischen Vorrichtungen Kontaktlinsen oder Intraokularlinsen oder Hydrogel-Hornhautimplantate sind.

**13.** Verfahren nach einem der Ansprüche 10 bis 12,

wobei Schritt (a) das Erzeugen eines gepulsten Laserstrahls mit einer Wellenlänge im Bereich von 1030 nm bis 1040 nm, einer Impulsbreite von weniger als 350 Femtosekunden und einer Wiederholungsrate von weniger als 20 MHz umfasst; und
wobei das Laserschreibsystem so betrieben wird, dass es eine Phasenverschiebung einer einzelnen Schreibschicht von mindestens 0,3 Wellen bei einer durchschnittlichen Leistung am Schreibkopf von weniger als 1500 mW und einer Scangeschwindigkeit von mehr als 100 mm/s induziert.

**14.** Verfahren nach Anspruch 13, wobei die ophthalmische Vorrichtung ein Hydrogelmaterial ist, das einen Vier-Photonen-Absorptionsparameter im Bereich von 1e-59 m$^6$ Wellen/(W$^4$ s) bis 3e-59 m$^6$ Wellen/(W$^4$ s) in der Gleichung:

$$\Delta\phi = \gamma \cdot \frac{P^4 \cdot NA^5}{\upsilon^3 \cdot \tau^3 \cdot S \cdot \lambda^7}$$

wobei $\Delta\phi$ eine geschriebene einschichtige Phasenverschiebung ist, $P$ eine mittlere Leistung des gepulsten Laserstrahls am Schreibkopf ist, $NA$ eine numerische Apertur des Schreibkopfs ist, v eine Laser-Wiederholrate ist, $\lambda$ eine Laser-Wellenlänge ist, $S$ eine Abtastgeschwindigkeit ist, $t$ *eine* Laser-Pulsdauer ist und $\gamma$ der Vier-Photonen-Absorptionsparameter ist.

## Revendications

**1.** Une méthode de conception d'un système d'écriture au laser pour modifier une pluralité de dispositifs ophtalmiques, la méthode comprenant :

(a) déterminer au moins une caractéristique matérielle des dispositifs ophtalmiques, déterminée sur une gamme de paramètres du système d'écriture laser ;
(b) déterminer au moins une caractéristique de conception du dispositif ophtalmique ; et
(c) en utilisant au moins le matériau déterminé et les caractéristiques de conception, configurer au moins un paramètre du système d'écriture au laser pour optimiser le débit du système d'écriture au laser, le système d'écriture au laser comprenant :

(i) un laser configuré pour générer un faisceau laser,
(ii) un séparateur configuré pour diviser le faisceau laser en une pluralité de sorties, et

(iii) une pluralité de têtes d'écriture, chaque tête d'écriture étant configurée pour diriger au moins une des sorties vers un dispositif ophtalmique afin d'inscrire une ou plusieurs modifications localisées de l'indice de réfraction dans le dispositif ophtalmique ;

dans lequel l'au moins une caractéristique de conception comprend un déphasage maximal conçu pour le dispositif ophtalmique à écrire dans le dispositif ophtalmique avec le système d'écriture laser sans causer de dommages optiques dans les dispositifs ophtalmiques ;

dans lequel l'au moins une caractéristique du matériau comprend au moins un premier seuil d'endommagement pour un déphasage optique induit d'une seule couche du dispositif ophtalmique à un premier taux de répétition laser, et un second seuil d'endommagement pour un déphasage optique induit d'une seule couche du dispositif ophtalmique à un second taux de répétition laser ; et

dans lequel l'optimisation du débit du système d'écriture au laser consiste à déterminer un nombre optimal de couches d'écriture pour modifier les dispositifs ophtalmiques en combinaison avec un nombre optimal de têtes d'écriture du système d'écriture au laser pour optimiser le débit du système d'écriture au laser et obtenir le déphasage maximal conçu pour le dispositif ophtalmique sans causer de dommages optiques dans les dispositifs ophtalmiques .

2. La méthode de la revendication 1, dans laquelle la gamme de paramètres du système d'écriture au laser utilisés pour déterminer l'au moins une caractéristique du matériau comprend au moins une gamme de puissance, une gamme de vitesse de balayage, une gamme de taux de répétition du laser et une gamme d'ouverture numérique de la lentille de mise au point.

3. La méthode de la revendication 2, dans laquelle la gamme de paramètres du système d'écriture au laser utilisés pour déterminer l'au moins une caractéristique du matériau comprend au moins deux des éléments suivants : la gamme de puissance, la gamme de vitesse de balayage, la gamme de taux de répétition du laser et l'ouverture numérique de la lentille de mise au point.

4. La méthode de la revendication 2 ou 3, dans laquelle les caractéristiques déterminées du matériau comprennent également au moins un paramètre d'ajustement d'un modèle quantitatif reliant un déphasage induit dans le dispositif ophtalmique à une configuration du système d'écriture laser.

5. La méthode de la revendication 4, dans laquelle les caractéristiques déterminées du matériau comprennent également un ordre multi photonique du modèle quantitatif, le modèle quantitatif comprenant de préférence un facteur de saturation.

6. La méthode de la revendication 5, dans laquelle le modèle quantitatif est un modèle de régime à deux photons comprenant :

$$\Delta\phi\left(P,v,S,NA\right):=\beta\cdot\left(\frac{P^2\cdot NA}{v\cdot\lambda^3\cdot S\cdot\tau}\right)$$

dans lequel $\Delta\phi$ est un déphasage écrit pour une seule couche, P est une puissance moyenne, $NA$ est une ouverture numérique, v est un taux de répétition du laser, $\lambda$ est une longueur d'onde du laser, S est une vitesse de balayage, $t$ est une durée d'impulsion, et $\beta$ est le paramètre d'ajustement ; ou

dans lequel le modèle quantitatif est un modèle de régime à trois photons comprenant :

$$\Delta\Phi:=\frac{\gamma\cdot P^3\cdot NA^3}{v^2\cdot\tau^2\cdot\lambda^5\cdot S}$$

dans lequel $\Delta\phi$ est un déphasage écrit pour une seule couche, $P$ est une puissance moyenne, $NA$ est une ouverture numérique, v est un taux de répétition du laser, $\lambda$ est une longueur d'onde du laser, S est une vitesse de balayage, $t$ est une durée d'impulsion, et $\gamma$ est le paramètre d'ajustement ; ou

dans lequel le modèle quantitatif est un modèle de régime à quatre photons comprenant :

$$\Delta\phi = \gamma \cdot \frac{P^4 \cdot NA^5}{v^3 \cdot \tau^3 \cdot S \cdot \lambda^7}$$

où $\Delta\phi$ un déphasage écrit d'une seule couche, $P$ est une puissance moyenne, $NA$ est une ouverture numérique, v est un taux de répétition du laser, $\lambda$ est une longueur d'onde du laser, $S$ est une vitesse de balayage, $\tau$ est une durée d'impulsion, et $\gamma$ est le paramètre d'ajustement.

7. La méthode de la revendication 5, dans laquelle le modèle quantitatif est un modèle de régime de photons N$^{th}$ comprenant :

$$\Delta\Phi := \frac{\gamma \cdot P^N \cdot NA^{2 \cdot N - 3}}{v^{N-1} \cdot \tau^{N-1} \cdot \lambda^{2 \cdot N - 1} \cdot S}$$

où $\Delta\phi$ est un déphasage écrit pour une seule couche, $P$ est une puissance moyenne, $NA$ est une ouverture numérique, v est un taux de répétition du laser, $\lambda$ est une longueur d'onde du laser, $S$ est une vitesse de balayage, $\tau$ est une durée d'impulsion, et $\gamma$ est le paramètre d'ajustement.

8. La méthode de la revendication 4, dans laquelle le modèle quantitatif comprend une somme de plusieurs modèles de régime multi photonique,

de préférence, le modèle quantitatif comprend

$$\Delta\Phi := \beta \cdot \frac{P^2 \cdot NA}{v \cdot \tau \cdot \lambda^3 \cdot S} + \gamma \cdot \frac{P^3 \cdot NA^3}{v^2 \cdot \tau^2 \cdot \lambda^5 \cdot S} + \delta \cdot \frac{P^4 \cdot NA^5}{v^3 \cdot \tau^3 \cdot \lambda^7 \cdot S}$$

dans lequel $\Delta\phi$ est un déphasage écrit pour une seule couche, $P$ est une puissance moyenne, $NA$ est une ouverture numérique, v est un taux de répétition du laser, $\lambda$ est une longueur d'onde du laser, $S$ est une vitesse de balayage, $t$ est une durée d'impulsion, $\beta$ est un paramètre d'ajustement du deuxième ordre, $\gamma$ est un paramètre d'ajustement du troisième ordre et $\delta$ est un paramètre d'ajustement du quatrième ordre.

9. La méthode de l'une des revendications 1 à 8, dans laquelle l'optimisation du débit du système d'écriture laser consiste à déterminer au moins l'un des éléments suivants : fréquence de répétition du laser, longueur d'onde du laser, largeur d'impulsion du laser et vitesse de balayage optimales pour optimiser le débit du système d'écriture laser ; et
de préférence, il s'agit en outre de déterminer une caractéristique d'espacement des lignes de l'appareil ophtalmique.

10. La méthode d'écriture au laser pour écrire des changements localisés d'indice de réfraction dans des dispositifs ophtalmiques avec un débit optimisé tout en obtenant un déphasage maximal conçu pour les dispositifs ophtalmiques sans causer de dommages optiques dans les dispositifs ophtalmiques, la méthode concevant un système d'écriture laser par la méthode de l'une quelconque des revendications 1 à 9, la méthode d'écriture au laser comprenant en outre :

(a) générer un faisceau laser pulsé dans le système d'écriture laser ;
(b) diviser le faisceau laser pulsé en une pluralité de sorties, au moins certaines des sorties étant chacune associées à l'une des têtes d'écriture du système d'écriture laser, parmi un nombre optimal de têtes d'écriture ; et
(c) à chacune des têtes d'écriture en nombre optimal, balayer le faisceau laser pulsé par rapport à un dispositif ophtalmique pour inscrire un nombre optimal de couches d'écriture ayant un ou plusieurs changements d'indice de réfraction dans le dispositif ophtalmique ;

dans lequel le nombre optimal de têtes d'écriture et le nombre optimal de couches d'écriture sont déterminés en combinaison pour optimiser le débit du système d'écriture laser tout en obtenant le déphasage maximal conçu pour les dispositifs ophtalmiques sans causer de dommages optiques dans les dispositifs ophtalmiques.

11. La méthode de la revendication 10, dans laquelle l'étape (a) consiste à générer un faisceau laser pulsé ayant une largeur d'impulsion inférieure à 350 femto secondes et une fréquence de répétition comprise entre 1 et 60 MHz ;

où, de préférence, le faisceau laser pulsé généré a une longueur d'onde comprise entre 340 nm et 1 100 nm ;

où, de préférence, le faisceau laser pulsé généré a une longueur d'onde comprise entre 515 nm et 520 nm, ou entre 1030 nm et 1040 nm, ou entre 400 nm et 410 nm, ou entre 795 nm et 805 nm ; et

où, de préférence, à l'étape (b), le faisceau laser est divisé en 2 à 64 sorties.

12. La méthode de la revendication 10 ou 11, dans laquelle les dispositifs ophtalmiques sont des lentilles de contact ou des lentilles intraoculaires, ou des implants cornéens en hydrogel.

13. La méthode de l'une des revendications 10 à 12,

l'étape (a) consiste à générer un faisceau laser pulsé d'une longueur d'onde comprise entre 1030 nm et 1040 nm, d'une largeur d'impulsion inférieure à 350 femto secondes et d'une fréquence de répétition inférieure à 20 MHz ; et

dans lequel le système d'écriture laser est utilisé pour induire un déphasage de la couche d'écriture unique d'au moins 0,3 onde à une puissance moyenne de la tête d'écriture inférieure à 1500 mW et à une vitesse de balayage supérieure à 100 mm/s.

14. La méthode de la revendication 13, dans laquelle le dispositif ophtalmique est un matériau hydrogel ayant un paramètre d'absorption de quatre photons compris entre 1e-59 m$^6$ ondes/(W$^4$ s) et 3e-59 m$^6$ ondes/(W$^4$ s) dans l'équation :

$$\Delta\phi = \gamma \cdot \frac{P^4 \cdot NA^5}{\upsilon^3 \cdot \tau^3 \cdot S \cdot \lambda^7}$$

où $\Delta\phi$ est un déphasage d'une seule couche écrite, $P$ est une puissance moyenne du faisceau laser pulsé au niveau de la tête d'écriture, $NA$ est une ouverture numérique de la tête d'écriture, v est un taux de répétition du laser, $\lambda$ est une longueur d'onde du laser, $S$ est une vitesse de balayage, $t$ est une durée d'impulsion du laser, et $\gamma$ est le paramètre d'absorption des quatre photons.

Figure 1

Figure 2(a)

Figure 2(b)

Figure 2(c)

Figure 2(d)

Figure 3(a)

Figure 3(b)

**Fringes**

Figure 3(c)

Figure 3(d)

Figure 4(a)

Figure 4(b)

Figure 4(c)

EP 3 867 003 B1

Figure 4(d)

Figure 5(a)

Figure 5(b)

Figure 6

Figure 7

Figure 8

Laser (12)

1

2

4

8 outputs (16)

50% beamsplitter

100% reflector

Figure 9

Figure 10

Figure 11

Figure 12

Figure 13

Figure 14

Figure 15

Figure 16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015040338 A1
- US 20090188901 A1
- US 20100228345 A1
- US 20160144580, Wayne H,. Knox
- US 8932352 B, Wayne H. Knox
- US 9144491 B, Wayne H. Knox